# EUROPEAN PATENT APPLICATION

(11) **EP 1 136 122 A1**
(43) Date of publication of application: **26.09.2001**
(21) Application number: 00961027.0
(22) Date of filing: 14.09.2000
(51) Int. Cl.: B01J 31/22, C07C 2/08, C08F 4/70, C08F 4/62, C08F 10/00, C08F 12/00

(54) **TRANSITION METAL CATALYSTS AND PROCESSES FOR PRODUCING $g(a)-OLEFIN AND VINYL COMPOUND POLYMER**

(30) Priority: 16.09.1999 JP 26256599; 12.11.1999 JP 32292899; 16.06.2000 JP 2000180875
(71) Applicant: IDEMITSU PETROCHEMICAL CO., LTD., Tokyo 130-0015 (JP)
(72) Inventor: SATO, Haruhito, Ichihara-shi, Chiba 299-0107 (JP); KURAMOTO, Masahiko, Ichihara-shi, Chiba 299-0107 (JP); WATANABE, Masami, Ichihara-shi, Chiba 299-0107 (JP)
(74) Representative: Hrabal, Ulrich
(86) International application number: JP0006317
(87) International publication number: WO0119513

(57) **Abstract**

A catalyst comprising (a) a complex of Group 8 to 10 transition metal of the Periodic Table, (b) clay, clay mineral or a ion-exchangeable layered compound, and (c) an amine compound or its adduct with Brφnsted acid; a catalyst comprising (a) a chelate complex of Group 4 to 6 transition metal of the Periodic Table or a complex of Group 8 to 10 transition metal of the Periodic Table, (b) clay, clay mineral or a ion-exchangeable layered compound, and (c) a quaternary ammonium salt; and a catalyst comprising (a) a complex of Group 4 to 6 transition metal of the Periodic Table or a complex of Group 8 to 10 transition metal of the Periodic Table and a co-catalyst component produced by contacting (b) clay, clay mineral or a ion-exchangeable layered compound, (c) an amine compound, its adduct with Brφnsted acid or a quaternary ammonium salt, and (d) an organosilane compound. Since these catalysts show high oligomerization activity of ethylene, α-olefins can be efficiently produced with low costs from ethylene. By using the catalysts, vinyl-terminated, linear α-olefins (oligomers) having a molecular weight of 10000 or less or polyolefins having a molecular weight exceeding 10000 can be also produced efficiently with low costs.

## Description

### Technical Field

The present invention relates to a catalyst for producing α-olefins and a process for producing α-olefins, more specifically, a catalyst capable of efficiently producing α-olefins with low costs and a process for producing α-olefins by oligomerization of ethylene using such a catalyst.

The present invention further relates to a catalyst for polymerizing olefins and a process for polymerizing olefins, more specifically, a catalyst exhibiting a high olefin-polymerization activity even in the absence of expensive aluminoxane co-catalysts and a process for polymerizing olefins using such a catalyst.

The present invention still further relates to a co-catalyst useful as a component of metallocene and other catalysts for polymerizing vinyl compounds, a catalyst for polymerizing vinyl compounds containing such a co-catalyst and a process for producing vinyl polymers using such a catalyst, more specifically, a co-catalyst for polymerizing vinyl compounds capable of efficiently producing vinyl polymers, a catalyst for polymerizing vinyl compounds and a process for producing vinyl polymers.

### Background Art

As a process for producing ethylene oligomers by polymerization of ethylene,-known is SHOP (Shell Higher Olefin Process) using a nickel complex. Although this process is industrially used in producing ethylene oligomers, it involves a drawback of a low activity.

Recently, proposed is a process in which ethylene is oligomerized to α-olefin using a transition metal complex as the main catalyst and an oxygen-containing organoaluminum compound such as aluminoxane, etc. or a boron-containing compound such as perfluorotetraphenyl borate, etc. as the co-catalyst. For example, European Patent No. 366212 proposes a process using a metallocene catalyst comprising a metallocene complex having Zr as the central atom and an aluminoxane. However, the activity per catalyst weight is low because the oxygen-containing compound such as aluminoxane should be used several hundred times by mole or more with respect to the main catalyst. In addition, the preparation of the boron-containing compound such as perfluorotetraphenyl borate is very difficult.

It has been know found that ethylene can be polymerized in the presence of an iron chelate complex (Chem. Commun., 1998, 849-850). This document reports that an iron chelate complex having a tridentate ligand bonding to the central metal via nitrogen atoms shows a high polymerization activity to ethylene when combinedly used with an aluminoxane co-catalyst, and the resultant ethylene oligomer is good in the terminal selectivity. However, the proposed process is still insufficient due to the use of expensive methylaluminoxane and the poor efficiency per catalyst.

Metallocene catalysts comprising a combination of a metallocene complex and an aluminoxane, developed as an olefin-polymerization catalyst, have been widely considered. However, to obtain a sufficiently high catalyst activity, expensive aluminoxane should be used excessively with respect to the metallocene complex. To eliminate this problem, Japanese Patent Application Laid-Open No. 5-25214 proposes to support the aluminoxane on clay mineral. As a specific example, this prior art teaches to support methylaluminoxane on smectite. However, the use of a large amount of methylaluminoxane is still necessary and the polymerization activity per aluminum is still insufficient. To make the clay mineral effective as a co-catalyst for an olefin-polymerization catalyst, the clay mineral is generally subjected to a treatment such as treatment with trialkylaluminum (Japanese Patent Application Laid-Open No. 5-301917), intercalation of organic substance (Japanese Patent Application Laid-Open No. 7-224106), treatment with a silane compound (Japanese Patent Application Laid-Open No. 11-106418). However, the clay minerals treated by the proposed methods are not dispersed stably in a slurry to result in a poor polymerization activity.

Accordingly, an object of the present invention is to provide a catalyst for producing α-olefins which exhibits a high oligomerization activity to ethylene without using expensive aluminoxane as the co-catalyst, and a process for producing α-olefins by oligomerization of ethylene using such a catalyst.

Another object of the present invention is to provide a novel olefin-polymerization catalyst which exhibits a high polymerization activity without using expensive aluminoxane as the co-catalyst, and a process for polymerizing olefins using such a catalyst.

Still another object of the present invention is to eliminate the above problems of the known metallocene catalyst using aluminoxane and provide a catalyst capable of efficiently producing vinyl polymers, particularly, vinyl polymers having an end vinyl group. It is also an object of the present invention to provide a novel co-catalyst component for producing vinyl polymers useful as a component of such a catalyst, and a process for producing the vinyl polymers.

### Disclosure of Invention

As a result of the extensive study in view of the above problems in the prior art, the inventors have found that a novel catalyst comprising (a) a complex of Group 8 to 10 transition metal of the Periodic Table, (b) a clay, clay mineral or ion-exchangeable layered compound, (c) an amine compound or its adduct with Brφnsted acid, and optionally (d) an organometallic compound is effective for oligomerizing ethylene to α-olefins.

The inventors have further found that a novel catalyst comprising (a) a chelate complex of Group 4 to 6 transition metal of the Periodic Table or a complex of Group 8 to 10 transition metal of the Periodic Table, (b) a clay, clay mineral or ion-exchangeable layered compound, (c) a quaternary ammonium salt, and optionally (d) an organometallic compound is effective for polymerization of olefins.

The inventors have still further found that a catalyst containing a co-catalyst component prepared by contacting (a) a clay, clay mineral or ion-exchangeable layered compound, (b) an amine compound, its adduct with Brφnsted acid or quaternary ammonium salt and (c) a silane compound is effective for producing vinyl polymers.

The present invention has been accomplished based on these findings.

Thus, in a first aspect of the present invention, there is provided a catalyst for producing α-olefins, comprising (a) a complex of Group 8 to 10 transition metal of the Periodic Table, (b) clay, clay mineral or a ion-exchangeable layered compound, and (c) an amine compound or its adduct with Brφnsted acid.

In a second aspect of the present invention, there is provided a process for producing α-olefins, comprising a step of oligomerizing ethylene in the presence of the catalyst for producing α-olefins mentioned above.

In a third aspect of the present invention, there is provided a catalyst for polymerizing olefins, comprising (a) a chelate complex of Group 4 to 6 transition metal of the Periodic Table or a complex of Group 8 to 10 transition metal of the Periodic Table, (b) clay, clay mineral or a ion-exchangeable layered compound, and (c) a quaternary ammonium salt.

In a fourth aspect of the present invention, there is provided a process for polymerizing olefins, comprising a step of oligomerizing ethylene in the presence of the catalyst for polymerizing olefins mentioned above.

In a fifth aspect of the present invention, there is provided a co-catalyst component for polymerizing vinyl compounds, produced by contacting (a) clay, clay mineral or a ion-exchangeable layered compound, (b) an amine compound, its adduct with Brφnsted acid or a quaternary ammonium salt, and (c) an organosilane compound.

In a sixth aspect of the present invention, there is provided a catalyst for polymerizing vinyl compounds, comprising (d) a complex of Group 4 to 6 transition metal of the Periodic Table or a complex of Group 8 to 10 transition metal of the Periodic Table and (e) the co-catalyst component mentioned above.

In a seventh aspect of the present invention, there is provided a process for producing polymers of vinyl compounds, comprising a step of polymerizing at least one vinyl compound selected from the group consisting of olefins, styrene, styrene derivatives, acrylic derivatives and vinyl esters of fatty acids in the presence of the catalyst for polymerizing vinyl compounds mentioned above.

### Best Mode for Carrying Out the Invention

### (1) First embodied catalyst

The first embodied catalyst of the present invention is a catalyst for producing α-olefins comprising (1-A) a complex of Group 8 to 10 transition metal of the Periodic Table, (1-B) a clay, clay mineral or ion-exchangeable layered compound (hereinafter may be simply referred to as "clay, etc."), and (1-C) an amine compound or its adduct with Brφnsted acid.

The catalyst for producing α-olefins may further contain (1-D) at least one organometallic compound selected from the group consisting of organoaluminum compound, organomagnesium compounds, organolithium compounds and organozinc compounds.

The term "α-olefin" referred to in the present invention is an oligomer having a molecular weight of 10,000 or less and an end vinyl group. The "α-olefin" is different, in its properties and application fields, from higher molecular-weight ethylene polymers showing properties typical for usual high-molecular polymers. Therefore, as compared with the catalyst for producing typical high-molecular polymers, the catalyst for producing α-olefins is required to have variant performance, and a catalyst for producing typical high-molecular polymer is not necessarily applicable to produce α-olefins.

The components (1-A) to (1-D) are specifically described below.

### Component (1-A)

This component is a so-called main catalyst and may be selected from a wide range of complexes of Group 8 to 10 transition metal of the Periodic Table.

Preferred complexe of Group 8 to 10 transition metal of the Periodic Table may include metal complexes represented by the following formulas (1) and (2):

L¹L²MX¹ ₘY¹ ₙ (1)

L¹L²L³MX¹ ₘY¹ ₙ (2).

In the formulas (1) and (2), M is a Group 8 to 10 transition metal of the Periodic Table, preferably iron, cobalt, nickel, palladium or platinum, and more preferably iron, cobalt or nickel.

Each of L¹ to L³ is a ligand capable of bonding to the transitional metal via a coordinating heteroatom. L¹ and L² of formula (1) or L¹ to L³ of formula (2) are preferably bonded to each other to form a chelate ligand. Examples of the coordinating heteroatoms include nitrogen, oxygen and sulfur, and nitrogen is preferable. The coordinating nitrogen is preferably bonded to carbon unsaturatedly, and more preferably forms -C=N-.

X¹ and Y¹ may be the same or different, and each represents a covalent- or ion-bonding group such as hydrogen; halogen such as fluorine, chlorine and iodine; C₁₋₂₀, preferably C₁₋₁₀ hydrocarbon group such as methyl, ethyl, n-propyl, isopropyl, cyclopentyl, cyclohexyl and cyclooctyl; C_{1-20,} preferably C₁₋₁₀ alkoxy such as methoxy, ethoxy, n-propoxy and isopropoxy; amino such as dimethylamino and diethylamino; C₁₋₂₀, preferably C₁₋₁₂ phosphorus-containing group such as diphenylphosphino; C₁₋₂₀, preferably C₁₋₁₂ slicon-containing group such as trimethylsilyl and trimethylsilylmethyl; and halogen-containing boride anion such as ⁻BF₄. Preferred are halogen and C₁₋₂₀ hydrocarbon group.

Each of m and n is 0 or a positive integer, and the sum of m and n is 0, 1, 2 or 3 depending on the valence of M.

Although not particularly limited, the transition metal complexes represented by the formula (1) are preferably those having a diimine ligand represented by the following formula (3): 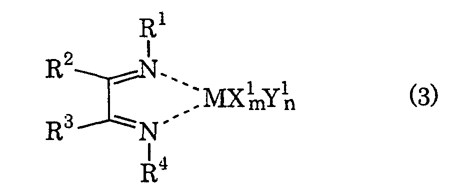 wherein M is a Group 8 to 10 transition metal of the Periodic Table; R¹ and R⁴ are each independently C₁₋₂₀ aliphatic hydrocarbon group or C₇₋₂₀ aromatic group having a hydrocarbon group on its aromatic ring; R² and R³ are each independently hydrogen or C₁₋₂₀ hydrocarbon group, and may be bonded together to from a ring; X¹ and Y¹ may be the same or different and are each a covalent- or ion-bonding group; and m and n are each 0 or a positive integer, the sum of m and n being 0, 1, 2 or 3 depending on the valence of M.

In the formula (3), M, X¹, Y¹, m and n are the same as defined in the formula (1). Specifically, M is particularly preferably nickel and X¹ and Y¹ are each preferably halogen or C₁₋₂₀ hydrocarbon group, and more preferably chlorine or methyl.

C₁₋₂₀ aliphatic hydrocarbon group for R¹ and R⁴ may include C₁₋₂₀ straight-chain or branched alkyl and C₃₋₂₀ cycloalkyl such as methyl, ethyl, n-propyl, isopropyl, n-butyl, isobutyl, s-butyl, t-butyl, pentyl, hexyl, octyl, decyl, tetradecyl, hexadecyl, octadecyl, cyclopentyl, cyclohexyl and cyclooctyl. Cycloalkyl may have a suitable substituent such as lower alkyl on its ring. Examples of C₇₋₂₀ aromatic group which has a hydrocarbon group on the aromatic ring include phenyl and naphthyl having one or more C₁₋₁₀ straight-chain or branched alkyls or cyclic alkyls on their aromatic rings. Preferred R¹ and R⁴ are aromatic groups having a hydrocarbon group on the aromatic rings, and 2,6-diisopropylphenyl is particularly preferable. R¹ and R⁴ may be the same or different from each other.

C₁₋₂₀ hydrocarbon group for R² and R³ may include C₁₋₂₀ straight-chain or branched alkyl, C₃₋₂₀ cycloalkyl, C₆₋₂₀ aryl, C₇₋₂₀ arylalkyl. Examples for C₁₋₂₀ straight-chain or branched alkyl and C₃₋₂₀ cycloalkyl are the same as those mentioned above. C₆₋₂₀ aryl may be phenyl, tolyl, xylyl, naphthyl, methylnaphthyl, etc. C₇₋₂₀ arylalkyl may be benzyl, phenethyl, etc. R² and R³ may be the same or different from each other, and may be bonded to each other to form a ring.

Specific examples of the complex represented by the formula (3) are shown below by the formulae [1] to [12].

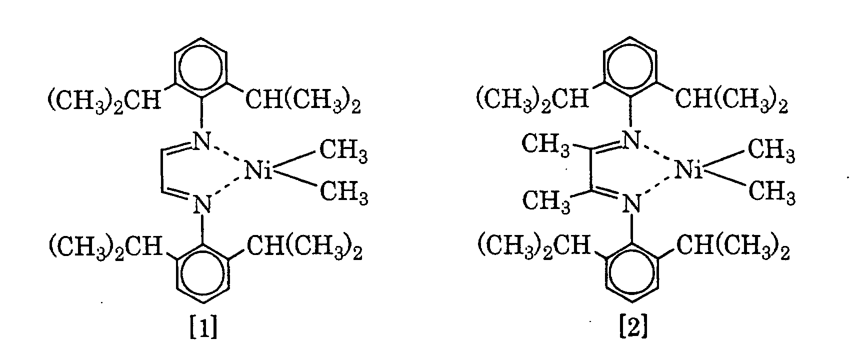

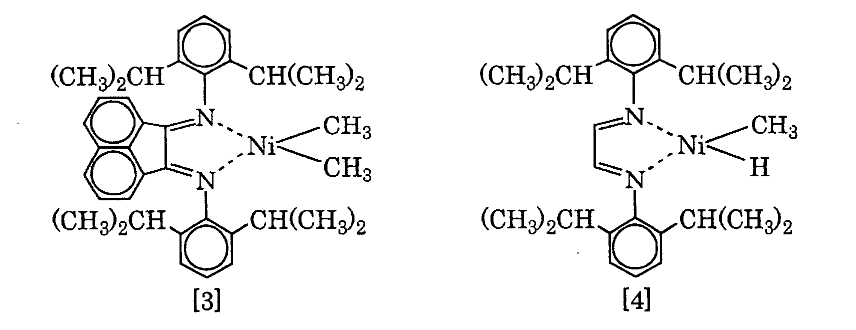

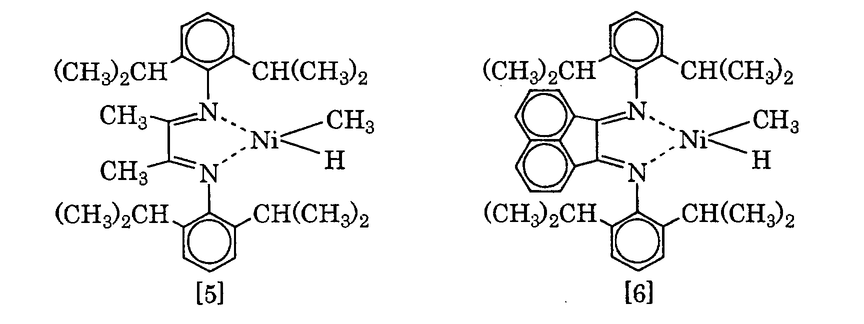

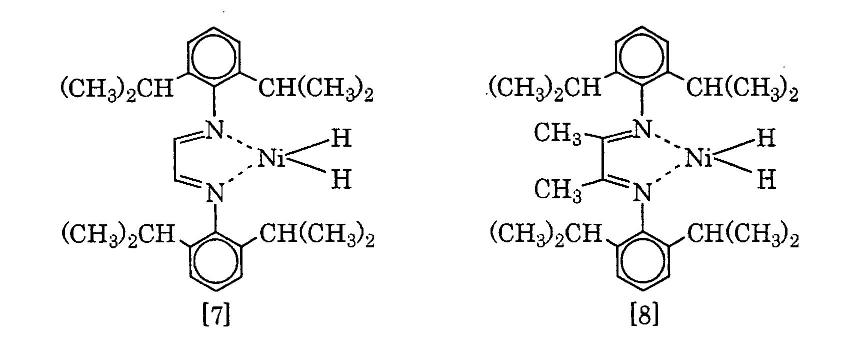

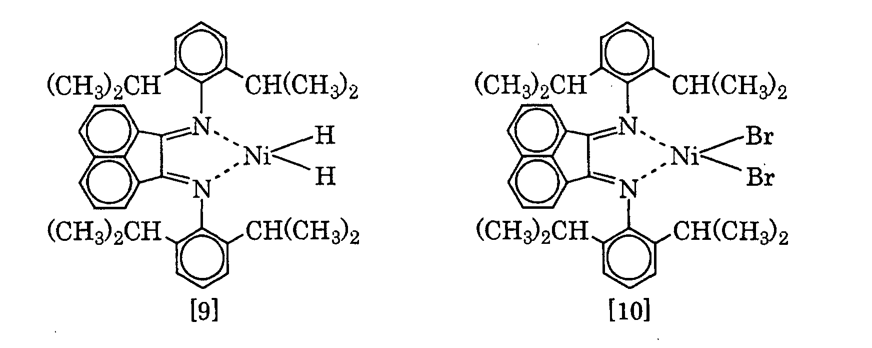

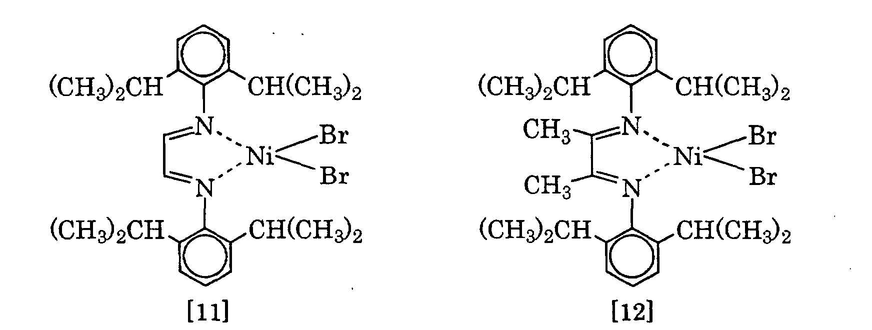

The transition metal complex represented by the formula (2) is preferably a nickel, iron or cobalt chelate complex having coordinating nitrogen atoms. Examples thereof are described in J. Am. Chem. Soc., 1998, 120, 4049-4050, Chem. Commun. 1998, 849-850, WO 98/27124, WO 99/02472 and WO 99/12981.

For example, the transition metal complex represented by the formula (2) may be a complex represented by the following formula (4): 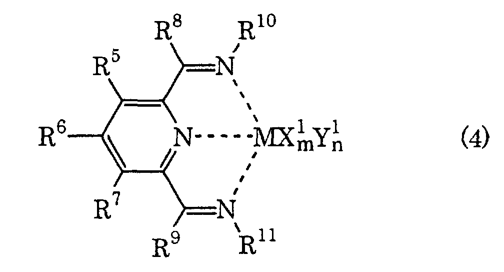 wherein M is a Group 8 to 10 transition metal of the Periodic Table; R⁵ to R¹¹, which may be bonded to each other to form a ring structure, are each independently hydrogen, halogen, hydrocarbon group, substituted hydrocarbon group or hetroatom-containing hydrocarbon group; X¹ and Y¹ may be the same or different and are each a covalent- or ion-bonding group; m and n are each 0 or a positive integer and the sum of m and n is 0, 1, 2 or 3 depending on the valence of M.

In the above formula (4), R⁵ to R¹¹ are each independently hydrogen, halogen, hydrocarbon group, substituted hydrocarbon group or heteroatom-containing hydrocarbon group. Halogen may include fluorine, chlorine, bromine and iodine. The hydrocarbon group may be C₁₋₃₀ hydrocarbon group, for example, C₁₋₃₀ straight-chain alkyl such as methyl, ethyl and n-propyl; C₃₋₃₀ branched alkyl such as isopropyl, s-butyl and t-butyl; C₃₋₃₀ alicyclic hydrocarbon group such as cyclopentyl and cyclohexyl; and C₆₋₃₀ aromatic hydrocarbon group such as phenyl and naphthyl. The substituted hydrocarbon group may be C₁₋₃₀ substituted hydrocarbon group derived from the hydrocarbon group mentioned above by substituting at least one hydrogen with a substituent such as hydrocarbon group, halogen and hetero atom-containing hydrocarbon group. The hydrocarbon substituent may be the same as the hydrocarbon group mentioned above. The hetero atom may be nitrogen, oxygen, sulfur, etc. The hetero atom-containing hydrocarbon group may be alkoxy represented by -OR, amino represented by -NR₂ or silyl represented by -SiR₃, wherein R is hydrocarbon group mentioned above.

M, X¹, Y¹, m and n in the formula (4) are the same as defined in the formula (2). Preferred M is iron, cobalt or nickel. Preferred X¹ and Y¹ are halogen and C₁₋₂₀ hydrocarbon group, and chlorine and methyl are more preferable.

Specific examples of the transition metal complex represented by the formula (4) may include iron or cobalt complexes having 2,6-diacetylpyridinebis(imine) ligand, 2,6-diformylpyridinebis(imine) ligand, 2,6-dibenzoylpyridinebis(imine) ligand, etc. Particularly preferred are iron or cobalt complexes having 2,6-diacetylpyridinebis(imine) ligand represented by the following formula (5): 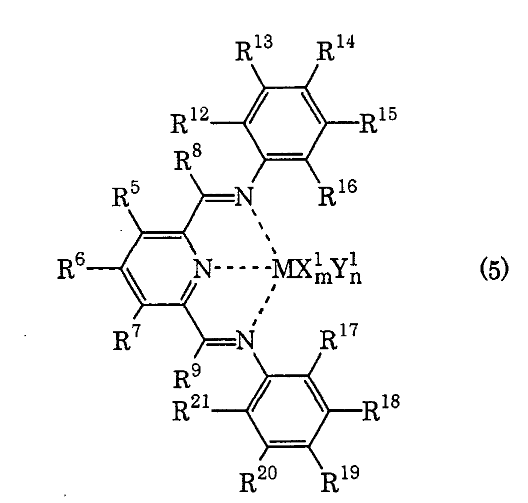 wherein M is a Group 8 to 10 transition metal of the Periodic Table; R⁵ to R⁹ and R¹² to R²¹ are each independently hydrogen, halogen, hydrocarbon group, substituted hydrocarbon group or hetero atom-containing hydrocarbon group and two adjacent groups of R¹² to R²¹ may be bonded to each other to form a ring; X¹ and Y¹ may be the same or different and are each covalent- or ion-bonding group; and m and n are each zero or a positive integer and the sum of m and n is 0, 1, 2 or 3 depending on the valence of M.

R⁵ to R⁹ and R¹² to R²¹ of the formula (5) are the same as R⁵ to R¹¹ of the formula (4).

R¹² may be a primary, secondary or tertiary carbon group. When R¹² is a primary carbon group, zero to two of R¹⁶, R¹⁷ and R²¹ may be a primary carbon group and the remainder thereof may be hydrogen. When R¹² is a secondary carbon group, zero or one of R¹⁶, R¹⁷ and R²¹ may be a primary or secondary carbon group and the remainder may be hydrogen. When R¹² is a tertiary carbon group, R¹⁶, R¹⁷ and R²¹ may be hydrogen.

Preferably, when R¹² is a primary carbon group, zero to two of R¹⁶, R¹⁷ and R²¹ is a primary carbon group and the remainder is hydrogen. When R¹² is a secondary carbon group, zero or one of R¹⁶, R¹⁷ and R²¹ is a primary or secondary carbon group and the remainder is hydrogen. When R¹² is a tertiary carbon group, R¹⁶, R¹⁷ and R²¹ are each hydrogen. Two adjacent groups of R¹² to R²¹ may be bonded to each other to form a ring.

M, X¹, Y¹, m and n in the formula (5) are the same as defined previously. Preferred M is iron, cobalt or nickel, and iron is particularly preferred. X¹ and Y¹ are preferably halogen (more preferably chlorine), C₁₋₂₀ hydrocarbon group (more preferably methyl) and C₁₋₂₀ silicon-containing group.

The following combination of the substituents of the formula (5) is preferable.

R⁸ and R⁹ are each methyl or hydrogen; and/or R⁵, R⁶ and R⁷ are all hydrogen; and/or R¹³, R¹⁴, R¹⁵, R¹⁸, R¹⁹ and R²⁰ are all hydrogen; and/or R¹⁶ and R²¹ are each independently methyl, ethyl, propyl or isopropyl, preferably both methyl or ethyl; and/or X¹ and Y¹ are each monovalent anion, preferably selected from halide, nitrite and hydrocarbon.

The following combinations of the substituents is also preferable. Namely, when R¹² is a primary carbon group, R¹⁶ is a primary carbon group and R¹⁷ and R²¹ are each hydrogen. Alternatively, when R¹² is a secondary carbon group, R¹⁶ is a primary or secondary carbon group, preferably a secondary carbon group and R¹⁷ and R²¹ are each hydrogen. When R¹² is a tertiary carbon group, R¹⁶, R¹⁷ and R²¹ are each hydrogen.

The following combinations of the substituents of the formula (5) are particularly preferable.
R⁸ = R⁹ = methyl, R¹³ = R¹⁴ = R¹⁵ = R¹⁸ = R¹⁹ = R²⁰ = hydrogen, and R¹⁶ = R²¹ = methyl;
R⁸ = R⁹ = methyl, R¹³ = R¹⁴ = R¹⁵ = R¹⁸ = R¹⁹ = R²⁰ = hydrogen, and R¹⁶ = R²¹ = ethyl;
R⁸ = R⁹ = methyl, R¹³ = R¹⁴ = R¹⁵ = R¹⁸ = R¹⁹ = R²⁰ = hydrogen, and R¹⁶ = R²¹ = isopropyl;
R⁸ = R⁹ = methyl, R¹³ = R¹⁴ = R¹⁵ = R¹⁸ = R¹⁹ = R²⁰ = hydrogen; and R¹⁶ = R²¹ = n-propyl;
R⁸ = R⁹ = methyl, R¹³ = R¹⁴ = R¹⁵ = R¹⁸ = R¹⁹ = R²⁰ = hydrogen, and R¹⁶ = R²¹ = chlorine; and
R⁸ = R⁹ = methyl, R¹³ = R¹⁴ = R¹⁵ = R¹⁸ = R¹⁹ = R²⁰ = hydrogen, and R¹⁶ = R²¹ = trifluoromethyl;

In any of the above combinations, X¹ and Y¹ are each preferably chlorine, bromine or nitrile compound, and more preferably chlorine.

The transition metal complex represented by the formula (5) can be produced, for example, by reacting a ketone compound represented by the following formula (6): 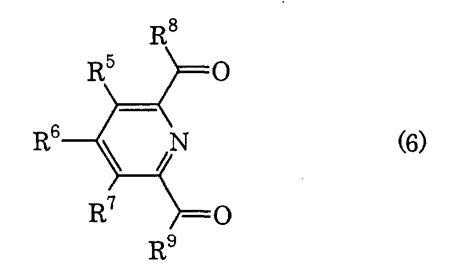 with an amine compound represented by H₂NR²² or H₂NR²³ wherein R²² and R²³
are 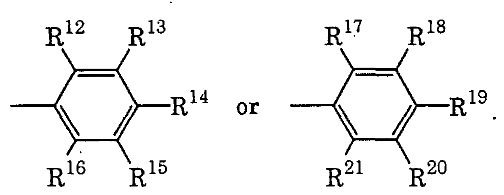 The reaction may be conducted in the presence of an organic acid such as formic acid as a catalyst. The compound from the reaction is then reacted with a halide of transition metal M to obtain the transition metal complex of the formula (5).

Any of the transition metal complexes of the formulas (1) and (2) may be used as the component (1-A), and preferred is the transition metal complex of the formula (2). Also, the transition metal complex may be used alone or in combination of two or more as the component (1-A).

### Component (1-B)

The component (1-B) is clay, clay mineral or ion-exchangeable layered compound. Clay is a substance composed of fine hydrous silicate minerals, and plastic when kneaded with a limited amount of water, hard when dried and sintered when burnt at high temperatures. The clay mineral is a hydrous silicate forming a substantial part of clay. Either of clay or clay minerals, which may be natural or synthesized, may be used for preparing the catalyst for producing α-olefins.

The ion-exchangeable layered compound is a compound having a layered crystalline structure comprising stacked parallel layers of atoms bonded each other by ion bonding, etc. Each layers are weakly bonded and ions contained therein are exchangeable. Some clay minerals are ion-exchangeable layered compounds.

Example of the clay mineral as the component (1-B) include phyllosilicate minerals such as phyllosilicic acid and a phyllosilicate. Natural phyllosilicates include smectite group such as montmorillonite, saponite, and hectorite, mica group such as illite and sericite, and mixed layered minerals of smectite group and mica group or mica group and vermiculite group. Synthesized phyllosilicate include TETRASILICON FLUORIDE MICA (Co-op Chemical Co., Ltd.), LAPONITE (Laporte Incustries, Ltd.) and SMECTON (Kunimine Industries, Ltd.). In addition, non-clay, ionic crystalline compound having a layered crystalline structure such as α-Zr(HPO₄)_{2, γ} -Zr(HPO₄)_{2,} α -Ti(HPO₄)₂ and _{γ} -Ti(HPO₄)₂ may be used.

Other usable clays and clay minerals not classified into the ion-exchangeable layered compound include bentonite clay with a lower content of montmorillonite, Kibushi clay or gairome clay containing montmorillonite with other major components, fibrous sepiolite or palygorskite, and amorphous or low crystalline allophane or imogolite.

The component (1-B) is preferably in the form of particle having a volume average particle size of 10 µm or less, more preferably 3 µm or less. The particle of the component (1-B) preferably has a size distribution in which the volume average particle size is 10 µm or less and the content of particles having a volume average particle size of 3.0 µm or less is 10 % by weight or more, more preferably a size distribution in which the volume average particle size is 10 µm or less and the content of particles having a volume average particle size of 1.5 µm or less is 10 % by weight or more. The volume average particle size and the content of particles of a given volume average particle size may be determined by a laser transmission particle size analyzer such as CIS-1 manufactured by Galai Production Ltd.

Of the component (1-B), those having a high capability of adsorbing the (1-C) amine compound or its adduct with Brφnsted acid described below or those having a high capability of producing an intercalation compound by the reaction with clay, etc. are preferable. For Example, clay and clay minerals are preferable. More specifically, the component (1-B) is preferably phyllosilicate minerals, more preferably smectite, and particularly preferably montmorillonite.

### Component (1-C)

The amine compound is preferred to be bulky. Assuming that the bulkiness is expressed by a ratio of the number of carbon atoms to the number of nitrogen atoms, the ratio is preferably 10 or more, more preferably 18 or more. The preferred ratio can be achieved by increasing the number of carbon atoms of the hydrocarbon group. When having the same number of carbon atoms, aromatic hydrocarbon groups are preferable to aliphatic hydrocarbon groups because an improved activity can be obtained. The amine compound is more preferred to have two or more aromatic hydrocarbon groups.

Examples of the amine compounds having a carbon atom number/nitrogen atom number ratio of 10 or more include aliphatic amines such as tributylamine, dicyclohexylamine, trioctylamine, bis(2-ethylhexyl)amine, tris(2-ethylhexyl)amine, tri-n-decylamine, tri-n-octylamine, tridodecylamine and N,N-di-n-octadecylmethylamine; benzylamines such as 4-benzylpiperazine, dibenzylamine and tribenzylamine; aromatic amines such as N,N-diethylaniline, N,N-dimethyl-1-naphthylamine, diphenylamine, triphenylamine, carbazole and N,N-dibenzylaniline; and heteroaromatic amines such as 2-benzylpyridine, 3-benzylpyridine, 4-benzylpyridine, 2-phenylpyridine, 3-phenylpyridine, 4-phenylpyridine and 1-n-octadecylpyrrole. Of the above amines, the benzylamines and the aromatic amines are preferable. More preferred are amine compounds, such as tribenzylamine, N,N-dibenzylaniline and 2-benzylpyridine, having two or more aromatic or heteroaromatic rings in their molecules.

The above amine compound may be treated with a Brφnsted acid to form an adduct of the amine compound and the Brφnsted acid prior to the use for preparing a clay-amine composite mentioned below. Examples of the adduct of the amine compound and the Brφnsted acid include adducts of the above amine compounds with a Brφnsted acid such as hydrochloric acid, sulfuric acid, etc.

### Component (1-D)

The component (1-D) is at least one organometallic compound selected from the group consisting of organoaluminum compounds, organomagnesium compounds, organolithium compounds and organozinc compounds, and the organoaluminum compounds are preferably used due to their low costs and easy availability. Examples of the organoaluminum compounds include trialkylaluminums such as trimethylaluminum, triethylaluminum, tripropylaluminum, triisobutylaluminum and tri-t-butylaluminum; halogen or alkoxy-containing alkylaluminums such as dimethylaluminum chloride, diethylaluminum chloride, dimethylaluminum methoxide and diethylaluminum ethoxide; and alumoxanes such as methylalumoxane, ethylalumoxane and isobutylalumoxane. Of the above, the trialkylaluminums are preferable, and triisobutylaluminum is more preferable.

The amount ratio between the component (1-A), the component (1-B), the component (1-C) and the optionally usable component (1-D) will be described.

The component (1-A) (transition metal complex) is used in an amount of 0.1 to 1000 µmol, preferably 1 to 100 µmol per unit weight (g) of the component (1-B) (clay, etc.). The component (1-C) (amine compound or its adduct with Brφnsted acid) is used in an amount of 0.001 to 2 mmol, preferably 0.01 to 1 mmol per unit weight (g) of the component (1-B). The component (1-D) (organometallic compound) may be optionally used in an amount of 0.01 to 100 mmol, preferably 0.1 to 10 mmol per unit weight (g) of the component (1-B). The component (1-D) may be used in an amount exceeding the above range, and the excessive portion of the component (1-D) can be removed from the catalyst system by washing a slurry suspension containing clay, etc. The amount of the component (1-C) (amine compound or its adduct with Brφnsted acid) is preferably less than the amount of the ion exchange capacity of the component (1-B). For example, since the ion exchange capacity of montmorillonite is 0.9 meq per unit weight (g), the amount of the component (1-C) to be used is preferred to be less than 0.9 meq.

The preparation method for the catalyst for producing α-olefins of the present invention is described below. In a most preferred method, the component (1-B) and the component (1-C) are contacted with each other in advance to obtain a clay-amine composite which is then contacted with the component (1-D) to remove impurities such as water contained in the clay-amine composite. The resultant clay-amine composite is then contacted with the component (1-A) to obtain the catalyst. When prepared in such a manner, the catalyst shows the highest activity and selectivity. More specifically, the component (1-C) is added to a suspension of the component (1-B) in 10 times or more of water and the mixture is stirred at room temperature, preferably room temperature to 100°C for 10 minutes or more, preferably 15 minutes or more, more preferably one hour or more, thereby obtaining a slurry, which is then filtered through a filter to obtain the clay-amine composite. It is preferable to add the component (1-C) in the form of aqueous solution in view of improving the contact efficiency in the mixture. A homogeneous aqueous solution of the component (1-C) can be prepared by adding acid such as hydrochloric acid, sulfuric acid and phosphoric acid. An alcohol such as methanol and ethanol may be further added, if desired. The separated clay-amine composite is dried to remove the moisture contained therein. The trace amount of the remaining impurities such as water is preferably removed by contacting the clay-amine composite with the component (1-D) under heating. The contacting treatment is preferably carried out in the same solvent as used in the subsequent polymerization process. Aliphatic hydrocarbons, aromatic hydrocarbons and halogenated hydrocarbons may be used as the solvent. Preferred solvents are aliphatic hydrocarbons such as butane, pentane, hexane, heptane and cyclohexane.

After such a pre-treatment, the clay-amine composite is contacted with the component (1-A). The contact time is 10 minutes or more, preferably 15 minutes or more, more preferably one hour or more, and most preferably 12 hours or more. The contact temperature may be room temperature to the boiling point of the solvent. These contact treatments may be carried out preferably in a stream of inert gas such as argon and nitrogen. The same solvent as used in the subsequent polymerization process is preferably used. The solvent is preferably aliphatic hydrocarbon such as butane, pentane, hexane, heptane and cyclohexane. In addition, the contact treatment is preferred to be carried out in the absence of a compound detrimental to catalyst, such as water and a compound having active hydrogen-containing group such as hydroxyl group, amino group, etc. Therefore, it is preferred to remove the compound having active hydrogen in advance from the contacting system using the component (1-D). The component (1-D) for this purpose may be used during either the preparation of the catalyst or the production of α-olefins.

Next, the process for producing α-olefins of the present invention will be described. In the process of the present invention, ethylene is oligomerized in the presence of the catalyst optionally containing the component (1-D). The method for oligomerization is not particularly limited and may be carried out by any known methods such as a solution reaction using a solvent, a liquid-phase non-solvent reaction using substantially no solvent, a vapor-phase reaction, etc. In addition, the oligomerization may be carried out in either continuous manner or batch-wise manner. As the solvent, if used, aliphatic hydrocarbon solvent such as butane, pentane, hexane, heptane and cyclohexane may be used. Of the above solvents, cyclohexane is particularly preferable because the deterioration of the product purity due to formation of alkylated by-product can be avoided as compared with using aromatic hydrocarbon solvent such as toluene. Known catalysts containing clay, etc. as their components show significant decrease with time in their polymerization activity when the solvent is changed from aromatic hydrocarbon solvent such as toluene to cyclohexane solvent. The combination of the catalyst for producing α-olefins of the present invention and cyclohexane as the solvent is advantageous because such a problem involved in prior art can be eliminated. The solvent may be used singly or in combination of two or more. When the solvent is used, the amount of catalyst in terms of the component (1-A) contained therein is generally 0.01 to 100 µmol, preferably 0.1 to 20 µmol per one liter of solvent in view of obtaining sufficient reactivity.

The oligomerization conditions are not specifically limited. The reaction temperature is generally -78 to 200°C, preferably room temperature to 150°C. The reaction pressure is generally ordinary pressure to 15 MPa·G, preferably ordinary pressure to 5 MPa·G. The molecular weight can be controlled by know methods, for example, by suitably selecting the temperature and pressure.

### (2) Second embodied catalyst

The second embodied catalyst comprises (2-A) a chelate complex of Group 4 to 6 transition metal of the Periodic Table or a complex of Group 8 to 10 transition metal of the Periodic Table, (2-B) clay, clay mineral or an ion-exchangeable layered compound (hereinafter may be collectively referred to as "clay, etc."), and (2-C) a quaternary ammonium salt. The second embodied catalyst may further contain, as an optional component, (2-D) at least one organometallic compound selected from the group consisting of organoaluminum compounds, organomagnesium compounds, organolithium compounds and organozinc compounds.

The components (2-A), (2-B), (2-C) and (2-D) will be described below more in detail.

### Component (2-A)

The so-called main catalyst is selected from the chelate complexes of Group 4 to 6 and Group 8 to 10 transition metal of the Periodic Table.

Examples of the Group 4 to 6 transition metal chelate complex include chelate complexes in which a ligand coordinates to a transition metal via two or more nitrogen atoms of aniline groups, which are represented by the following formula: 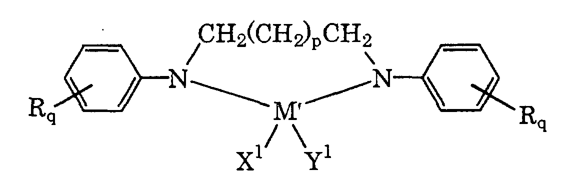 wherein M' is a Group 4 to 6 transition metal of the Periodic Table, R groups may be the same or different and are each C₁₋₂₀ alkyl, X¹ and Y¹ may be the same or different and are each a covalent- or ion-bonding group, p is an integer from 0 to 2, and q is an integer from 0 to 5.

M' is a Group 4 to 6 transition metal of the Periodic Table such as hafnium, niobium, molybdenum, tungsten, etc., and preferably titanium and zirconium. R groups may be the same or different and are each C₁₋₂₀ alkyl such as methyl, ethyl, n-propyl, isopropyl, etc. X¹ and Y¹ are the same as defined in the formulae (1) and (2). Suffix "p" is an integer from 0 to 2, preferably 1, and "q" is an integer from 0 to 5, preferably 0 to 3.

Specific examples of the above chelate complex having aniline ligands include the following MaConville-type titanium chelate complexes.

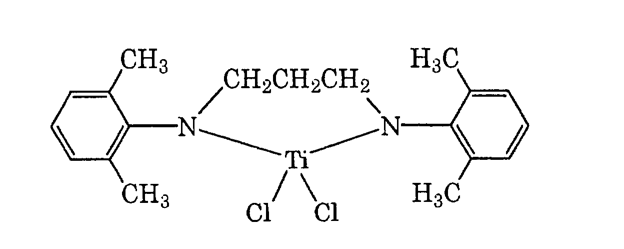

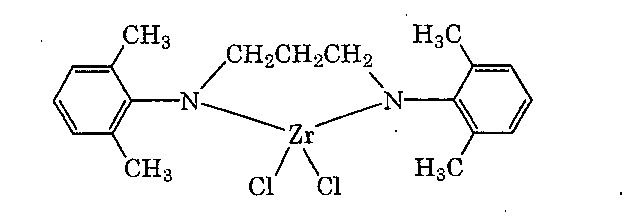

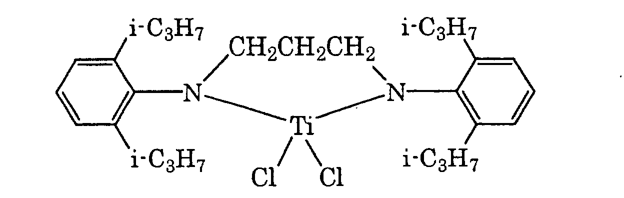

In addition to the above compounds, the corresponding transition metal compounds having zirconium, hafnium, niobium, molybdenum or tungsten, etc. instead of titanium may be usable. Further, in any of the transition metal compounds mentioned above, chlorine atom or atoms may be substituted by bromine, iodine, hydrogen, methyl, phenyl, etc.

The complex of Group 8 to 10 transition metal of the Periodic Table usable in the second embodied catalyst is the same as the component (1-A) of the first embodied catalyst.

The component (2-A) may be either the chelate complex of Group 4 to 6 transition metal of the Periodic Table or the complex of Group 8 to 10 transition metal of the Periodic Table, and preferably the complex of Group 8 to 10 transition metal of the Periodic Table. The complex of Group 8 to 10 transition metal of the Periodic Table may be the transition metal complex represented by either the formula (1) or the formula (2), and preferably the transition metal complex represented by the formula (2). Particularly preferred are coordinating nitrogen atom-containing iron, cobalt or nickel chelate complexes. The transition metal complex may be used alone or in combination of two or more as the component (2-A).

### Component (2-B)

The same clay, clay mineral and ion-exchangeable layered compound as the component (1-B) may be used as the component (2-B).

Of the component (2-B), those having a high capability of adsorbing (2-C) the quaternary ammonium salt described below or those having a high capability of producing an intercalation compound by the reaction with clay, etc. are preferable. For Example, clay and clay minerals are preferable. More specifically, the component (2-B) is preferably phyllosilicate minerals, more preferably smectite, and particularly preferably montmorillonite. Tetrasilicon fluoride mica is preferable as the synthesized phyllosilicate.

### Component (2-C)

The component (2-C) is a quaternary ammonium salt such as a quaternary alkylammonium salt, a quaternary arylammonium salt, a quaternary arylalkylammonium salt, a quaternary benzylammonium salt and a heteroaromatic ammonium salt, although not specifically limited thereto. Examples of the quaternary alkylammonium salt include tetra-n-propylammonium chloride, tetrabutylammonium chloride, dimethyldicyclohexylammonium chloride, methyltri-n-octylammonium chloride, methyltris(2-ethylhexyl)ammonium chloride, methyltri-n-decylammonium chloride, methyltri-n-dodecylammonium chloride and dimethyldi-n-octadecylammonium dichloride: Examples of the quaternary arylammonium salt include tetraphenylammonium chloride. Examples of the quaternary arylalkylammonium salt include phenyltrimethylammonium chloride, dimethyldiphenylammonium chloride and methyltriphenylammonium chloride. Examples of the quaternary benzylammonium salt include dimethyldibenzylammonium chloride, methyltribenzylammonium chloride and dimethylbenzylammonium chloride. Examples of the heteroaromatic ammonium salt include N-methyl-2-benzylpyridinium chloride, N-methyl-3-benzylpyridinium chloride, N-methyl-4-benzylpyridinium chloride, N-methyl-2-phenylpyridinium chloride, N-methyl-3-phenylpyridinium chloride and N-methyl-4-phenylpyridinium chloride. Bromides, fluorides and iodides corresponding to the above chlorides may be used as the quaternary ammonium salts. In the quaternary ammonium salt, the ratio of the number of carbon atoms to the number of nitrogen atoms is preferably 8 or more. More preferred are a quaternary ammonium salt having at least one aromatic ring-containing group such as the quaternary benzylammonium salt, quaternary arylammonium salt and quaternary arylalkylammonium salt; and a quaternary ammonium salt having two or more alkyl groups having 6 or more carbon atoms such as dimetyldicyclohexylammonium chloride, methyltri-n-octylammonium chloride, methyltris(2-ethylhexyl)ammonium chloride, methyltri-n-decylammonium chloride, methyltri-n-dodecylammonium chloride and dimetyldi-n-octylammonium chloride.

### Component (2-D)

The component (2-D) is at least one organometallic compound selected from the group consisting of the same organoaluminum compounds, organomagnesium compounds, organolithium compounds and organozinc compounds as mentioned for the component (1-D).

In the second embodied catalyst, the component (2-A) (transition metal complex) is used in an amount of 0.1 to 1000 µmol, preferably 1 to 100 µmol per unit weight (g) of the component (2-B) (clay, etc.). The component (2-C) (quaternary ammonium salt) is used in an amount of 0.001 to 2 mmol, preferably 0.01 to 1 mmol per unit weight (g) of the component (2-B). The component (2-D) (organometallic compound) may be optionally used in an amount of 0.01 to 100 mmol, preferably 0.1 to 10 mmol per unit weight (g) of the component (2-B). The component (2-D) may be used in an amount exceeding the above range, and the excessive component (2-D) can be removed from the catalyst system by washing a slurry suspension containing clay, etc. The amount of the component (2-C) is preferably less than the ion exchange capacity of the component (2-B). For example, since the ion exchange capacity of montmorillonite is 0.9 meq per unit weight (g), the amount of the component (2-C) to be used is preferred to be less than 0.9 meq.

The second embodied catalyst can be prepared in the same manner as in the first embodied catalyst except for using the component (2-C) instead of the component (1-C).

Next, the olefin polymerization of the present invention will be described. In the olefin polymerization of the present invention, olefins are polymerized in the presence of the catalyst optionally containing the component (2-D). Olefins may be ethylene or α-olefin such as propylene, butene-1, octene-1, etc., and preferably ethylene. The method for polymerization is not particularly limited and may be carried out by any known methods such as a solution reaction using a solvent, a liquid-phase non-solvent reaction using substantially no solvent, a vapor-phase reaction, etc. The solution reaction is preferable. In addition, the polymerization may be carried out in either continuous manner or batch-wise manner. As the solvent, if used, aliphatic hydrocarbon solvent such as butane, pentane, hexane, heptane and cyclohexane may be used. Of the above solvents, cyclohexane is particularly preferable because the deterioration of the product purity due to formation of alkylated by-product can be effectively avoided as compared with using aromatic hydrocarbon solvent such as toluene. Known catalysts containing clay, etc. as their components show significant decrease with time in their polymerization activity when the solvent is changed from aromatic hydrocarbon solvent such as toluene to cyclohexane solvent. The combination of the olefin polymerization catalyst of the present invention and cyclohexane as the solvent is advantageous because such a problem involved in prior art can be eliminated. The solvent may be used singly or in combination of two or more. When the solvent is used, the amount of catalyst in terms of the component (2-A) contained therein is generally 0.01 to 100 µmol, preferably 0.1 to 20 µmol per one liter solvent in view of obtaining sufficient reactivity.

The polymerization conditions are not specifically limited. The reaction temperature is generally -78 to 200°C, preferably room temperature to 150°C. The reaction pressure is generally ordinary pressure to 15 MPa·G, preferably ordinary pressure to 5 MPa·G. The molecular weight can be controlled by know methods, for example, by suitably selecting the temperature and pressure.

By the olefin polymerization using the catalyst of the present invention, vinyl-terminated α-olefins (oligomer) having a molecular weight of 10000 or less or polyolefins having a molecular weight of 10000 or more can be efficiently produced with low costs.

### (3) Third embodied catalyst

The third embodied catalyst for polymerizing vinyl compounds comprises (3-A) a complex of Group 4 to 6 or Group 8 to 10 transition metal of the Periodic Table, (3-B) a novel co-catalyst component for polymerizing vinyl compounds, and optionally (3-C) an organoaluminum compound.

Each component will be described below.

### Component (3-A)

The complex of Group 4 to 6 transition metal of the Periodic Table of the third embodied catalyst is the same Group 4 to 6 transition metal chelate complex as those for the component (2-A) or a compound listed in the following (1) to (10):
(1) Non-bridged transition metal compounds having two conjugated five-membered ligands such as bis(cyclopentadienyl) titanium dichloride, bis(methylcyclopentadienyl) titanium dichloride, bis(dimethylcyclopentadienyl) titanium dichloride and bis(trimethylcyclopentadienyl) titanium dichloride;
(2) Bridged transition metal compounds having two conjugated five-membered ligands which are linked by an alkylene bridge, such as methylenebis(indenyl) titanium dichloride, ethylenebis(indenyl) titanium dichloride, methylenebis(indenyl) titanium chlorohydride, ethylenebis(indenyl)methyl titanium chloride, ethylenebis(indenyl)methoxychloro titanium, ethylenebis(indenyl) titanium diethoxide and ethylenebis(indenyl)dimethyl titanium;
(3) Bridged transition metal compounds having two conjugated five-membered ligands which are linked by a silylene bridge, such as dimethylsilylenebis(indenyl) titanium dichloride, dimethylsilylenebis(4,5,6,7-tetrahydroindenyl) titanium dichloride, dimethylsilylenebis(2-methylindenyl) titanium dichloride, dimethylsilylenebis(2,4-dimethylindenyl) titanium dichloride, dimethylsilylene(2,4-dimethylcyclopentadienyl) (3',5'-dimethylcyclopentadienyl) titanium dichloride and phenylmethylsilylenebis(indenyl) titanium dichloride;
(4) Bridged transition metal compounds having two conjugated five-membered ligands which are linked by a germanium, aluminum, boron, phosphorus, or nitrogen-containing hydrocarbylene bridge, such as dimethylgermylenebis(indenyl) titanium dichloride, dimethylgermylene(cyclopentadienyl)(fluorenyl) titanium dichloride, methylalumylenebis(indenyl) titanium dichloride, phenylalumylenebis(indenyl) titanium dichloride, phenylphosphylenebis(indenyl) titanium dichloride, and ethylborenebis(indenyl) titanium dichloride;
(5) Transition metal compounds having one conjugated five-membered ligand, such as pentamethylcyclopentadienyl-bis(phenyl)amino titanium dichloride, indenyl-bis(phenyl)amino titanium dichloride, pentamethylcyclopentadienyl-bis(trimethylsilyl)amino titanium dichloride and pentamethylcyclopentadienylphenoxy titanium dichloride;
(6) Doubly bridged transition metal compounds having two conjugated five-membered ligands which are doubly linked by two bridges, such as (1,1'-dimethylsilylene)(2,2'-isopropylidene)-bis(cyclopentadienyl) titanium dichloride, (1,1'-dimethylsilylene)(2,2'-dimethylsilylene)-bis(cyclopentadienyl) titanium dichloride, (1,1'-dimethylsilylene)(2,2'-isopropylidene)-bis(cyclopentadienyl)dimethyl titanium and (1,1'-dimethylsilylene)(2,2'-isopropylidene)-bis(cyclopentadienyl)dibenzyl titanium;
(7) Transition metal compounds such as cyclopentadienyl titanium trichloride, methylcyclopentadienyl titanium trichloride, dimethylcyclopentadienyl titanium trichloride, trimethylcyclopentadienyl titanium trichloride, and tetramethylcyclopentadienyl titanium trichloride;
(8) Transition metal compounds such as 4,5,6,7-tetrahydroindenyl titanium trichloride and 2-methylindenyl titanium trichloride;
(9) Transition metal compounds such as octahydrofluorenyl titanium trichloride; and
(10) Transition metal compounds having one conjugated five-membered ligand, such as pentamethylcyclopentadienyl titanium trimethoxide and pentamethylcyclopentadienyl titanium trichloride.

The complex of Group 8 to 10 transition metal of the Periodic Table of the third embodied catalyst is the same as those for the component (1-A). In the third embodied catalyst, the transition metal complexes of the formula (5) having the following combinations of R⁵ to R⁹ and R¹² to R²¹ are particularly preferable.
R⁸ = R⁹ = R¹² = R²¹ = methyl, and R⁵ = R⁶ = R⁷ = R¹³ = R¹⁴ = R¹⁵ = R¹⁶ = R¹⁷ = R¹⁸ = R¹⁹ = R²⁰ = hydrogen;
R⁸ = R⁹ = methyl, R⁵ = R⁶ = R⁷ = R¹³ = R¹⁴ = R¹⁵ = R¹⁶ = R¹⁷ = R¹⁸ = R¹⁹ = R²⁰ = hydrogen, and R¹² = R²¹ = ethyl;
R⁸ = R⁹ = methyl, R⁵ = R⁶ = R⁷ = R¹³ = R¹⁴ = R¹⁵ = R¹⁶ = R¹⁷ = R¹⁸ = R¹⁹ = R²⁰ = hydrogen, and R¹² = R²¹ = isopropyl;
R⁸ = R⁹ = methyl, R⁵ = R⁶ = R⁷ = R¹³ = R¹⁴ = R¹⁵ = R¹⁶ = R¹⁷ = R¹⁸ = R¹⁹ = R²⁰ = hydrogen, and R¹² = R²¹ = n-propyl;
R⁸ = R⁹ = R¹² = R¹⁴ = R¹⁹ = R²¹ = methyl, and R⁵ = R⁶ = R⁷ = R¹³ = R¹⁵ = R¹⁶ = R¹⁷ = R¹⁸ = R²⁰ = hydrogen;
R⁸ = R⁹ = methyl, R⁵ = R⁶ = R⁷ = R¹³ = R¹⁴ = R¹⁵ = R¹⁶ = R¹⁷ = R¹⁸ = R¹⁹ = R²⁰ = hydrogen, and R¹² = R²¹ = chlorine; and
R⁸ = R⁹ = methyl, R⁵ = R⁶ = R⁷ = R¹³ = R¹⁴ = R¹⁵ = R¹⁶ = R¹⁷ = R¹⁸ = R¹⁹ = R²⁰ = hydrogen, and R¹² = R²¹ = trifluoromethyl.

The component (3-A) is preferably a complex of Group 8 to 10 transition metal of the Periodic Table which may be represented by the formula (1) or (2). Particularly preferred are iron, cobalt or nickel chelate complexes which contain coordinating nitrogen atoms. The transition metal complex may be used alone or in combination of two or more.

### Component (3-B)

The component (3-B) is a novel co-catalyst component for polymerizing vinyl compounds, and can be prepared by contacting (3-B1) clay, clay mineral or ion-exchangeable layered compound, (3-B2) an amine compound, an adduct of an amine compound and Brφnsted acid or a quaternary ammonium salt, and (3-B3) an organosilane compound.

### Component (3-B1)

The same clay, clay mineral and ion-exchangeable layered compound as used for the component (1-B) of the first embodied catalyst may be used as the component (3-B1) of the co-catalyst component.

The component (3-B1) is preferably a silicon-containing, ion-exchangeable layered compound such as phyllosilicic acid minerals and mica group minerals. Preferred phyllosilicic acid minerals are smectite group minerals such as montmoiillonite, saponite, etc. Montmorillonite may be called as refined bentonite or crude bentonite according to its composition. Mica group minerals may be exemplified by tetrasilicon fluoride mica known as a synthetic mica. Tetrasilicon fluoride mica is classified into a non-swelling mica and a swelling mica, and the swelling mica is preferably used in the present invention. The smectite group minerals and mica group minerals are particularly preferable as the component (3-B1) because these minerals improve the polymerization activity when used as a component of a catalyst for polymerizing vinyl compounds.

### Component (3-B2)

The component (3-B2) is an amine compound such as a primary alkylamine, secondary alkylamine and tertiary alkylamine; an adduct of the amine compound and Brφnsted acid; or a quaternary ammonium salt. Preferred are the tertiary alkylamine, its adduct with Brφnsted acid and the quaternary ammonium salt because these compounds improve the polymerization activity when used as a component of a catalyst for polymerizing vinyl compounds.

Examples of the tertiary alkylamine include alkylamines such as trimethylamine, triethylamine, tri-n-propylamine, tri-i-propylamine, tri-n-butylamine, dicyclohexylmethylamine and cyclohexyldimethylamine; alkenylamines such as vinyldiethylamine, allyldiethylamine, cyclohexenyldimethylamine, divinylethylamine, diallylmethylamine and dicyclohexenylmetylamine; arylalkylamines such as diphenylmethylamine, phenyldiethylamine, phenyldipropylamine and naphthyldimethylamine; and tribenzylamine. Of the above tertiary alkylamines, preferred are those in which a ratio of the number of carbon atoms to the number of nitrogen atoms (hereinafter may be referred to as "C/N ratio") is 8 or more. For example, the C/N ratio is 6 for triethylamine and 12 for tri-n-butylamine. Adducts of tertiary alkylamine and Brφnsted acid can be obtained by adding Brφnsted acid such as hydrochloric acid, sulfuric acid, etc. to the tertiary alkylamines recited above.

The quaternary ammonium salts of the component (3-B2) are the same as those for the component (2-C).

The component (3-B2) is generally used 0.001 to 2 mmol, preferably 0.01 to 1 mmol per unit weight (g) of the component (3-B1).

### Component (3-B3)

The organosilane compound is represented, although not particularly limited, by the following formula (7):

R²⁶ ᵣSiX₄₋ᵣ (7)

wherein R²⁶ is hydrogen or a group having a carbon or silicon atom which is directly bonded to Si, X is halogen or a group having an oxygen or nitrogen atom which is directly bonded to Si, r is an integer from 1 to 3, and a plurality of R²⁶ groups or X groups, if any, may be the same or different from each other.

The organosilane compound of the formula (7) includes polynuclear polysiloxane, polysilazane and bissilyl compound represented by the formula (8):

R²⁶ ₜX₃₋ₜSi(CH₂)ₛSiR²⁶ ₜX₃₋ₜ (8)

wherein s is an integer from 1 to 10, t is an integer from 1 to 3, and R²⁶ and X is as defined in the formula (7).

Examples of the organosilane compounds of the formula (7) include trialkylsilyl chlorides such as trimethylsilyl chloride, triethylsilyl chloride, triisopropylsilyl chloride, t-butyldimethylsilyl chloride, t-butyldiphenylsilyl chloride and phenethyldimethylsilyl chloride; dialkylsilyl dichlorides such as dimethylsilyl dichloride, diethylsilyl dichloride, diisopropylsilyl dichloride, di-n-hexylsilyl dichloride, dicylohexylsilyl dichloride, docosylmethylsilyl dichloride, bis(phenethyl)silyl dichloride, methylphenethylsilyl dichloride, diphenylsilyl dichloride, dimesitylsilyl dichloride and ditolylsilyl dichloride; alkylsilyl trichlorides such as methylsilyl trichloride, ethylsilyl trichloride, isopropylsilyl trichloride, t-butylsilyl trichloride, phenylsilyl trichloride and phenethylsilyl trichloride; and silyl halides obtained by substituting chlorine of the above compounds with another halogen. Other examples include hydride-containing silanes such as dimethylchlorosilane, (N,N-dimethylamino)dimethylsilane and diisobutylchlorosilane; alkylsilyl hydroxides such as trimethylsilyl hydroxide, triethylsilyl hydroxide, triisopropylsilyl hydroxide, t-butyldimethylsilyl hydroxide, phenethyldimethylsilyl hydroxide, dicyclohexylsilyl dihydroxide and diphenylsilyl dihydroxide; and polysilanols known as a peralkylpolysiloxypolyol.

Examples of the compounds of the formula (8) include bissilyl compounds such as bis(methyldichlorosilyl)methane, 1,2-bis(methyldichlorosilyl)ethane, bis(methyldichlorosilyl)octane and bis(triethoxysilyl)ethane. The polynuclear polysiloxane may be cyclic polysiloxanes such as 1,3,5,7-tetramethylcyclotetrasiloxane, 1,3,5,7-tetraethylcyclotetrasiloxane and
1,3,5,7-tetramethyl-1,3,5,7-tetraphenylcyclotetrasiloxane; and straight-chain polysiloxanes such as 1,1,5,5-tetraphenyl-1,3,3,5-tetramethyltrisiloxane. Examples of the polysilazane include disilazanes such as bis(trimethylsilyl)amide, bis(triethylsilyl)amide, bis(triisopropylsilyl)amide, bis(dimethylethylsilyl)amide, bis(diethylmethylsilyl)amide, bis(dimethylphenylsilyl)amide, bis(dimethyltolylsilyl)amide and bis(dimethylmenthylsilyl)amide.

Preferred organosilane compounds are those represented by the formula (7) or (8) wherein R²⁶ is alkyl, benzyl or aromatic group and X is chlorine or oxygen-containing group. The organosilane compound may be used alone or in combination of two or more.

The contact of the component (3-B3) (organosilane compound) and the component (3-B1) (clay, etc.) is effectively conducted in the presence of water. It can be assumed that water makes the coarse particles of clay, etc. finely dispersed and changes the layered structure of clay, thereby enhancing the contact efficiency of the organosilane compound with clay, etc. Namely, water increases the distance between crystalline layers of clay, etc. to promote the reaction between the organosilane compound and clay, etc. It should be noted that the present invention is not restricted by the above assumption.

The organosilane compound is used 0.001 to 1000 mmol, preferably 0.01 to 100 mmol in terms of silicon atom per unit weight (g) of the component (3-B1).

In view of obtaining an improved polymerization activity, preferred co-catalysts for polymerizing vinyl compounds of the present invention are those obtained by contacting (3-B1) an ion-exchangeable, silicon-containing layered compound such as smectite group minerals and mica group minerals, (3-B2) a tertiary alkylamine, its adduct with Brφnsted acid or a quaternary ammonium salt each having a C/N ratio of 8 or more, and (3-B3) an organosilane compound of the formula (7) or (8) wherein R²⁶ is alkyl, benzyl or aromatic group, X is halogen or oxygen-containing group, and r or s is 1 or 2.

The preparation method of the co-catalyst for polymerizing vinyl compounds is not strictly limited, and basically includes the following two methods. In one of the methods, clay, etc. are dispersed in water to prepare an aqueous colloidal solution of clay, into which the organosilane compound is added. The resultant mixture is stirred under heating and then the tertiary alkylamine, its salt with Brφnsted acid or the quaternary ammonium salt is added to the mixture to prepare the co-catalyst in the form of slurry. Since the co-catalyst is easily separated from water in the slurry, this method is advantageous because the co-catalyst can be collected by a simple filtration. In the other method, clay, etc. are pre-treated with the tertiary alkylamine, its salt with Brφnsted acid or the quaternary ammonium salt in water. The clay, etc. thus treated are then dispersed in an aprotic solvent such as hexane and toluene, to which the organosilane compound is added.

In both the methods, the mixing ratio of the component (3-B2) (amine compound, etc.) and the component (3-B3) (organosilane compound) is suitably determined so that the amount of each component per unit weight (g) of the component (3-B1) is within the range defined above.

### Component (3-C)

The organoaluminum compound as the component (3-C) is represented by the following formula (9):

R²⁴ ᵥAlQ₃₋ᵥ (9)

wherein R²⁴ is C₁₋₁₀ alkyl, Q is hydrogen, C₁₋₂₀ alkoxy, C₆₋₂₀ aryl or halogen and v is an integer from 1 to 3.

Examples of the compound of the formula (9) include trimethylaluminum, triethylaluminum, triisopropylaluminum, triisobutylaluminum, dimethylaluminum chloride, diethylaluminum chloride, methylaluminum dichloride, ethylaluminum dichloride, dimetyhlaluminum fluoride, diisobutylaluminum hydride, diethylaluminum hydride and ethylaluminum sesquichloride.

The organoaluminum compound may be used alone or in combination of two or more.

Other organoaluminum compounds may be aluminum oxy compounds such as a chain aluminoxane represented by the following formula (10): 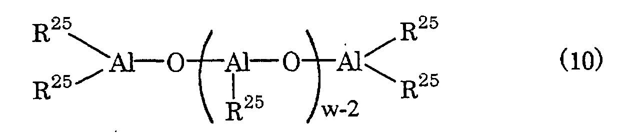 wherein R²⁵ is C_{1-20,} preferably C₂₋₁₂ hydrocarbon group such as alkyl, alkenyl, aryl and arylalkyl or halogen, w is an average degree of polymerization and usually an integer from 2 to 50, preferably from 2 to 40, and R²⁵ groups may be the same or different from each other,
and a cyclic aluminoxane represented by the following formula (11): 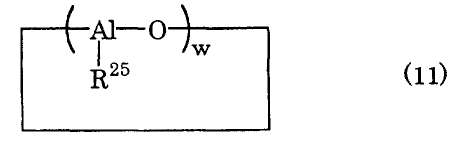 wherein R²⁵ and w are the same as defined in the formula (10).

Examples of the aluminoxane include ethylaluminoxane and isobutylaluminoxane.

The aluminoxane can be prepared, for example, by contacting an alkylaluminum with a condensation agent such as water. The contacting method is not particularly restricted and may be carried out according to known methods such as (1) a method where a solution of an organoaluminum compound in an organic solvent is contacted with water, (2) a method where an organoaluminum compound is added to the polymerization system and subsequently water is added to the polymerization system, and (3) a method where crystal water of metal salt or water adsorbed by inorganic or organic substances are reacted with an organoaluminum compound.

The aluminoxane may be soluble or insoluble, preferably soluble to hydrocarbon solvents. The content of the remaining organoaluminum compound is preferably 10% by weight or less, more preferably 3 to 5% by weight or less, particularly preferably 2 to 4% by weight or less when measured by ¹H-NMR. Such an aluminoxane is preferable because a larger amount thereof can be supported on carriers (high supporting ratio). In addition, non-supported aluminoxane can be easily recycled and reused because it is soluble to hydrocarbon solvents. Further, since such an aluminoxane is stable in its properties, no particular treatment is required for its use. Still further, polyolefins having improved average particle size and particle size distribution (called generically as morphology) can be obtained. A content of the remaining organoaluminum compound exceeding 10% by weight reduces the supporting ratio to decrease the polymerization activity.

Such an aluminoxane can be obtained, for example, by a method where an aluminoxane solution is evaporated to dryness by distilling off the solvent under reduced pressure while heating (dry-up method). In the dry-up method, the removal of the solvent is carried out preferably at 80°C or lower, more preferably at 60°C or lower.

The component insoluble to hydrocarbon solvent can be removed from the aluminoxane, for example, by the spontaneous sedimentation of the insoluble component and the subsequent decantation. The removal by centrifugation is also usable. To ensure sufficient removal of the insoluble component, the solution of the soluble component is preferably filtered through G5 glass filter, etc. in nitrogen stream. Since the gel component increases with time, the aluminoxane thus obtained should be used preferably within 48 hours from its preparation, more preferably immediately after its preparation. The concentration of the aluminoxane in the hydrocarbon solvent is not strictly limited, and preferably 0.01 to 10 mol in terms of aluminum atom per one liter of the hydrocarbon solvent.

Examples of the hydrocarbon solvent include aromatic hydrocarbon such as benzene, toluene, xylene, cumene and cymene; aliphatic hydrocarbon such as pentane, hexane, heptane, octane, decane, dodecane, hexadecane and octadecane; alicyclic hydrocarbon such as cyclopentane, cyclohexane, cyclooctane and methyhlcyclopentane; and petroleum fraction such as naphtha, kerosene and light gas oil.

The above aluminoxane may be used alone or in combination of two or more.

The catalyst for polymerizing vinyl compounds may be prepared by contacting clay, etc. which are treated by the amine compound and organosilane compound, i.e., the co-catalyst component for polymerizing vinyl compounds, with the component (3-A) (transition metal complex) optionally after added with the component (3-C) (organoaluminum compound), although not restricted thereto. The contact time depends on the kinds of the transition metal complex, and generally 10 minutes to several days, preferably 10 minutes to several hours. The amount of the component (3-A) is 0.1 to 1000 µmol, preferably 1 to 50 µmol, and the amount of the optional component (3-C) is preferably 20 mmol or less, each based on unit weight (g) of the co-catalyst component for polymerizing vinyl compounds.

In the process of the present invention, vinyl compound are polymerized in the presence of the above catalyst optionally containing the component (3-D) which is the same organometallic compound selected from the group consisting of organoaluminum compounds, organomagnesium compounds, organolithium compounds and organozinc compounds as defined for the component (1-D). The component (3-D) may be contained generally 0.01 to 100 mmol, preferably 0.1 to 10 mmol per unit weight (g) of the component (3-B1) (clay, etc.).

Examples of the vinyl compounds include olefins, styrene, styrene derivatives, acrylic derivatives and vinyl esters of fatty acids.

Olefins are not strictly limited, and preferably ethylene and α-olefin having 3 to 20 carbon atoms. Examples of such α-olefins include straight-chain or branched α-olefins such as 1-butene, 1-pentene, 1-hexene, 1-heptene, 1-octene, 1-nonene, 1-decene, 4-phenyl-1-butene, 6-phenyl-1-hexene, 3-methyl-1-butene, 4-methyl-1-butene, 3-methyl-1-pentene, 4-methyl-1-hexene, 5-methyl-1-hexene, 3,3-dimethyl-1-pentene, 3,4-dimethyl-1-pentene, 4,4-dimethyl-1-pentene and vinylcyclohexane; halogenated α-olefins such as hexafluoropropene, tetrafluoroethylene, 2-fluoropropene, fluoroethylene, 1,1-difluoroethylene, 3-fluoropropene, trifluoroethylene and 3,4-dichloro-1-butene; and cyclic olefins such as cyclopenetene, cyclohexene, norbornene, 5-methylnorbornene, 5-ethylnorbornene, 5-propylnorbornene, 5,6-dimethylnorbornene and 5-benzylnorbornene. Examples of the styrene derivatives include alkylstyrenes such as p-methylstyrene, p-ethylstyrene, p-propylstyrene, p-isopropylstyrene, p-butylstyrene, p-t-butylstyrene, p-phenylstyrene, o-methylstyrene, o-ethylstyrene, o-propylstyrene, o-isopropylstyrene, m-methylstyrene, m-ethylstyrene, m-isopropylstyrene, m-butylstyrene, mesitylstyrene, 2,4-dimethylstyrene, 2,5-dimethylstyrene and 3,5-dimethylstyrene; alkoxystyrenes such as p-methoxystyrene, o-methoxystyrene and m-methoxystyrene; halogenated styrenes such as p-chlorostyrene, m-chlorostyrene, o-chlorostyrene, p-bromostyrene, m-bromostyrene, o-bromostyrene, p-fluorostyrene, m-fluorostyrene, o-fluorostyrene and o-methyl-p-fluorostyrene; trimethylsilylstyrene; vinyl benzoate; and divinylbenzene. Examples of the acrylic derivatives include ethyl acrylate, butyl acrylate, methyl methacrylate and ethyl methacrylate.

Examples of the vinyl esters of fatty acids include vinyl acetate, isopropenyl acetate and vinyl acrylate.

In the process of the present invention, the vinyl compound may be used alone or in combination of two or more. When the copolymerization is intended, two or more of the above olefins may be combined arbitrarily.

In the process of the present invention, the above olefins may be copolymerized with another monomer exemplified by chain diolefins such as butadiene, isoprene, 1,4-pentadiene and 1,5-hexadiene; polycyclic olefins such as norbornene, 1,4,5,8-dimetano-1,2,3,4,4a,5,8,8a-octahydronaphthalene and 2-norbornene; cyclic diolefins such as norbornadiene, 5-ethylidenenorbornene, 5-vinylnorbornene and dicyclopentadiene; and unsaturated esters such as ethyl acrylate and methyl methacrylate.

The vinyl compound is preferably ethylene, propylene and styrene, and more preferably ethylene. The method for polymerizing vinyl compounds is not particularly limited and may be carried out by any known methods such as slurry polymerization, solution polymerization, vapor-phase polymerization, bulk polymerization and suspension polymerization, preferably by solution polymerization. In addition, the polymerization may be carried out in either continuous manner or batch-wise manner. As the solvent, if used, aliphatic hydrocarbon solvent such as different butane, pentane, hexane and heptane isomers and cyclohexane may be used. Of the above solvents, cyclohexane is particularly preferable because the deterioration of the product purity due to formation of alkylated by-product can be effectively avoided as compared with using aromatic hydrocarbon solvent such as toluene. The solvent may be used singly or in combination of two or more. When the solvent is used, the amount of catalyst in terms of the component (3-A) contained therein is generally 0.01 to 1000 µmol, preferably 0.1 to 500 µmol per one liter solvent in view of obtaining sufficient reactivity.

The polymerization conditions are not specifically limited. The reaction temperature is generally -78 to 200°C, preferably room temperature to 150°C. The reaction pressure is generally ordinary pressure to 15 MPa·G, preferably ordinary pressure to 5 MPa·G. The molecular weight can be controlled by know methods, for example, by suitably selecting the temperature and pressure.

By the polymerization of vinyl compound using the third embodied catalyst of the present invention, vinyl-terminated α-olefins (oligomer) having a number average molecular weight of 10000 or less or polyolefins having a number average molecular weight of 10000 or more can be efficiently produced with low costs.

The present invention will be described in more detail with reference to the following examples. However, it should be noted that the following examples are only illustrative and not intended to restrict the scope of the present invention thereto.

### Example 1

(1) Preparation of co-catalyst solution A
   Into a 10-liter flack containing 4 liter of distilled water, 10 g of Na-montmorillonite (BEN-GEL, available from Hojun Yoko, Co., Ltd.) were slowly added under stirring. After the addition was completed, stirring was continued for two hours at room temperature to prepare a clay-water colloidal solution. After heating the clay-water colloidal solution to 80°C, was added little by little a solution consisting of 2.19 g (8 mmol) of N,N-dibenzylaniline, 4 ml of conc. hydrochloric acid (35 to 37% concentration) and 40 ml of ethyl alcohol. After the addition, the mixture was stirred for two hours at the same temperature and for two hours at 100°C. After the colloidal solution was changed to clay slurry by flocculation, the slurry was filtered under heating through a pressure filter. The separated solid product was vacuum-dried at room temperature to obtain 12.8 g of clay-amine composite.
   Into a 2-liter flask containing 10 g of the clay-amine composite, 250 ml of 0.5 mol/liter toluene solution of triisobutylaluminum (TIBA) were added at room temperature After heating to 100°C, the mixture was stirred for one hour at the same temperature and cooled to obtain a clay slurry, which was then added with one liter of dry toluene and allowed to stand. The supernatant was discarded by a cannula and the slurry was washed with toluene repeatedly. Finally, the volume of the slurry was adjusted to 500 ml with toluene to obtain the co-catalyst solution A (clay-amine composite content: 20 mg/ml).
(2) Preparation of catalyst solution A
   Into 20 ml of toluene, was suspended 0.088 g (200 µmol) of a pyridinebisimine iron complex, [2,6-[(2,4-C₆H₃Me₂)N=C(Me)]₂C₅H₃N]FeCl₂, which was prepared according to the method described in J. Am. Chem. Soc., 1998, 4049 and Chem. Commun., 1998, 849, thereby preparing a complex slurry A (complex content: 10 µmol/ml). In a Schlenk tube, 2.5 ml of the co-catalyst solution A and 0.05 ml of the complex slurry A were mixed and stirred for one hour at room temperature to prepare a catalyst solution A.
(3) Oligomerization
   Into a deaerated 1.6-liter autoclave of 75°C, 400 ml of dry cyclohexane, 1 ml of toluene solution of tetraisobutyldialuminoxane ([(CH₃)₃CHCH₂]₂AlOAl[CH₂CH(CH₃)₂]₂) (Al content: 1.0 mol/liter) and all of the catalyst solution A prepared above were successively added in nitrogen stream. After heated to 80°C, ethylene was pumped into the autoclave to keep the reaction pressure at 0.8 MPa·G. After 30 minutes of starting the introduction of ethylene, the supply of ethylene was stopped and the reaction liquid was rapidly cooled by cooling water. After cooling and pressure release, 49.0 g (total yield) of the reaction product containing 7.9 g of polymer were obtained. The total polymerization activity and the oligomerization activity were 3510 kg/gFe/h and 2940 kg/gFe/h, respectively. The total yield is the amount subtracting the amount of the solvent from the amount of the reaction mixture recovered from the oligomerization. The recovered reaction mixture was pressure-filtered, and the solid matter on the filter was dried at 80°C for 4 hours under reduced pressure and weighed to obtain the amount of the polymer.

### Comparative Example 1

(1) Preparation of co-catalyst solution B
   By changing the method of preparing the co-catalyst solution A in Example 1 as shown below, the co-catalyst solution B was prepared.
   Into a 2-liter flask, 10 g of thoroughly dehydrated Na-montmorillonite (BEN-GEL, available from Hojun Yoko Co., Ltd.) were placed under nitrogen stream, to which 250 ml of 0.5 mol/liter toluene solution of triisobutylaluminum (TIBA) were added at room temperature. The mixture was heated to 100°C and stirred at the same temperature for one hour. After cooling, the clay slurry thus obtained was added with one liter of dry toluene and allowed to stand. The supernatant was discarded by a cannula and the slurry was washed with toluene repeatedly. Finally, the volume of the slurry was adjusted to 500 ml with toluene to obtain the co-catalyst solution B (clay content: 20 mg/ml).
(2) Preparation of catalyst solution B
   The catalyst solution B was prepared in the same manner as in Example 1(2) except for using the co-catalyst solution B prepared above instead of the co-catalyst solution A.
(3) Oligomerization
   The oligomerization of ethylene was conducted in the same manner as in Example 1(3) except for using the catalyst solution B instead of the catalyst solution A. The ethylene absorption was not detected even after 30 minutes of the ethylene supply, and the analysis of the recovered solution showed no production of oligomer and polymer.

### Example 2

The oligomerization of ethylene was conducted in the same manner as in Example 1(3) except for using toluene as the polymerization solvent instead of cyclohexane. The total yield was 27.1 g containing 0.8 g of polymer. The total polymerization activity and the oligomerization activity were 1940 kg/gFe/h and 1880 kg/gFe/h, respectively.

### Example 3

The oligomerization of ethylene was conducted in the same manner as in Example 1(3) except for changing the polymerization temperature from 80°C to 50°C. The total yield was 111.6 g containing 14.2 g of polymer. The total polymerization activity and the oligomerization activity were 7990 kg/gFe/h and 6980 kg/gFe/h, respectively.

### Example 4

(1) Preparation of co-catalyst solution C
   In the same manner as in Example 1(1) except for using 2.30 g (8 mmol) of tribenzylamine instead of 2.19 g (8 mmol) of N,N-dibenzylaniline, the co-catalyst solution C (clay-amine composite content: 20 mg/liter) was prepared.
(2) Preparation of catalyst solution C
   The catalyst solution C was prepared in the same manner as in Example 1(2) except for using the co-catalyst solution C instead of the co-catalyst solution A.
(3) Oligomerization
   The oligomerization of ethylene was conducted in the same manner as in Example 1(3) except for using the catalyst solution C instead of the catalyst solution A and changing the polymerization temperature from 80°C to 50°C. The total yield was 94 g containing 14.2 g of polymer. The total polymerization activity and the oligomerization activity were 6730 kg/gFe/h and 5720 kg/gFe/h, respectively.

### Example 5

(1) Preparation of co-catalyst solution D
   A clay-water colloidal solution was prepared in the same manner as in Example 1(1) except for using a different type of Na-montmorillonite (KUNIPIA F, available from Kunimine Kogyo Co., Ltd.) instead of Na-montmorillonite (BEN-GEL, available from Hojun Yoko Co., Ltd.). After heating the clay-water colloidal solution to 60°C, was added little by little a solution consisting of 0.968 g (8 mmol) of N,N-dimethylaniline, 4 ml of conc. hydrochloric acid (35 to 37% concentration) and 40 ml of water. After the addition, the mixture was stirred for two hours at the same temperature. After the colloidal solution was changed to clay slurry by flocculation, the slurry was filtered under heating through a pressure filter. The separated solid product was vacuum-dried at room temperature to obtain 10.2 g of clay-amine composite.
   Into a 2-liter flask, 10 g of the clay-amine composite were placed and then treated with triisobutylaluminum (TIBA) in the same manner as in Example 1(1) to prepare the co-catalyst solution D (clay-amine composite content: 20 mg/ml).
(2) Preparation of catalyst solution D
   The catalyst solution D was prepared in the same manner as in Example 1(2) except for using 5.0 ml of the co-catalyst solution D instead of 2.5 ml of the co-catalyst solution A and using the complex slurry A (complex content: 10 µmol/ml) in an amount of 0.1 ml instead of 0.05 ml.
(3) Oligomerization
   The oligomerization of ethylene was conducted in the same manner as in Example 1(3) except for using the catalyst solution D instead of the catalyst solution A, and using toluene as the polymerization solvent instead of cyclohexane. The total yield was 32.1 g containing 7.8 g of polymer. The total polymerization activity and the oligomerization activity were 1150 kg/gFe/h and 870 kg/gFe/h, respectively.

### Example 6

(1) Preparation of co-catalyst solution E
   A clay-amine composite was prepared in the same manner as in Example 5(1) except for using Na-montmorillonite (BEN-GEL, available from Hojun Yoko Co., Ltd.) instead of Na-montmorillonite (KUNIPIA F, available from Kunimine Kogyo Co., Ltd.), and using 0.129 g (1.2 mmol) of 2,6-dimethylpyridine instead of 0.968 g (8 mmol) of N,N-dimethylaniline. The clay-amine composite thus obtained was treated with TIBA to obtain a co-catalyst solution E (clay-amine composite content: 20 mg/ml).
(2) Preparation of catalyst solution E
   The catalyst solution E was prepared in the same manner as in Example 5(2) except for using the co-catalyst solution E instead of the co-catalyst solution D.
(3) Oligomerization
   The oligomerization of ethylene was conducted in the same manner as in Example 5(3) except for using the catalyst solution E instead of the catalyst solution D. The total yield was 44.4 g containing 3.8 g of polymer. The total polymerization activity and the oligomerization activity were 1590 kg/gFe/h and 1450 kg/gFe/h, respectively.

### Example 7

The oligomerization of ethylene was conducted in the same manner as in Example 4(3) except for changing the polymerization temperature to 80°C. The absorption rates of ethylene after 5 minutes, 10 minutes and 15 minute of the oligomerization were 4.3 liter/min, 2.5 liter/min and 1.5 liter/min, respectively. The relative absorption rates with respect to the absorption rate after 5 minutes are shown in Table 1.

**Table 1**

| Polymerization Time | Relative Absorption Rate |
|---|---|
| 5 minutes | 100 |
| 10 minutes | 58 |
| 15 minutes | 35 |

### Example 8

(1) Preparation of co-catalyst solution F
   Into a 2-liter flack containing one liter of distilled water, 2.5 g of Na-montmorillonite (BEN-GEL, available from Hojun Yoko, Co., Ltd.) were slowly added under stirring. After the addition was completed, stirring was continued for two hours at room temperature to prepare a clay-water colloidal solution. After heating the clay-water colloidal solution to 60°C, was added an aqueous solution of 0.496 g (2 mmol) of benzyldimethylphenylammonium chloride in 100 ml of water. After the addition, the mixture was stirred for one hour at the same temperature. The slurry thus obtained was filtered under heating through a pressure filter. The separated solid product was vacuum-dried at room temperature to obtain 2.9 g of clay-quaternary ammonium salt composite.
   Into a 300-ml flask containing 10 g of the clay-quaternary ammonium salt composite, 25 ml of 0.5 mol/liter toluene solution of triisobutylaluminum (TIBA) were added at room temperature After heating to 100°C, the mixture was stirred for one hour at the same temperature and cooled to obtain a clay slurry, which was then added with 250 ml of dry toluene and allowed to stand. The supernatant was discarded by a cannula and the slurry was washed with toluene repeatedly. Finally, the volume of the slurry was adjusted to 50 ml with toluene to obtain the co-catalyst solution F (content of clay-quaternary ammonium salt composite: 20 mg/ml).
(2) Preparation of catalyst solution F
   Into 20 ml of toluene, was suspended 0.088 g (200 µmol) of a pyridinebisimine iron complex, [2,6-[(2,4-C₆H₃Me₂)N=C(Me)]₂C₅H₃N]FeCl₂, which was prepared according to the method described in J. Am. Chem. Soc., 1998, 4049 and Chem. Commun., 1998, 849, thereby preparing a complex slurry A (complex content: 10 µmol/ml). In a Schlenk tube, 5.0 ml of the co-catalyst solution F and 0.2 ml of the complex slurry A were mixed and stirred for one hour at room temperature to prepare a catalyst solution F.
(3) Polymerization of ethylene
   Into a 1.6-liter autoclave of 50°C, 400 ml of dry cyclohexane, 0.5 ml of toluene solution of triisobutylaluminum (Al content: 1.0 mol/liter) and all of the catalyst solution F prepared above were successively added in nitrogen stream. Then, ethylene was pumped into the autoclave to keep the reaction pressure at 0.8 MPa • G. After 30 minutes of starting the introduction of ethylene, the supply of ethylene was stopped and the reaction liquid was rapidly cooled by cooling water. After cooling and pressure release, 100.7 g (total yield) of the reaction product containing 1.58 g of solid cyclohexane insolubles, i.e., polymer and 99.12 g of cyclohexane solubles, i.e., oligomer were obtained. The total polymerization activity and the polymerization activity for the cyclohexane solubles were 1800 kg/gFe/h and 1780 kg/gFe/h, respectively. ¹³C-NMR measurement showed that the polymer obtained was a vinyl-terminated polymer. Gas chromatographic analysis showed that the cyclohexane solubles contained 98 % by weight or more of a vinyl-terminated, linear α-olefin (oligomer having a carbon number of 8 to 18). In the gas chromatographic analysis, OV-1 column (60 m) was used for determining the amount of oligomer and Ultra-2 column (50 m) was used for determining the purity.

### Comparative Example 2

(1) Preparation of co-catalyst solution G
   Into a 300-ml flask, 2.5 g of thoroughly dehydrated Na-montmorillonite (BEN-GEL, available from Hojun Yoko Co., Ltd.) were placed under nitrogen stream, to which 250 ml of 0.5 mol/liter toluene solution of triisobutylaluminum (TIBA) were added at room temperature. The mixture was heated to 100°C and stirred at the same temperature for one hour. After cooling, the clay slurry thus obtained was added with 250 ml of dry toluene and allowed to stand. The supernatant was discarded by a cannula and the slurry was washed with toluene repeatedly. Finally, the volume of the slurry was adjusted to 50 ml with toluene to obtain the co-catalyst solution G (clay content: 20 mg/ml).
(2) Preparation of catalyst solution G
   The catalyst solution G was prepared in the same manner as in Example 8(2) except for using the co-catalyst solution G prepared above instead of the co-catalyst solution F.
(3) Polymerization of ethylene
   The polymerization of ethylene was conducted in the same manner as in Example 8(3) except for using the catalyst solution G instead of the catalyst solution F. The ethylene absorption was not detected even after 30 minutes of the ethylene supply, and the analysis of the recovered solution showed no production of oligomer and polymer.

### Example 9

(1) Preparation of catalyst solution H
   Into 20 ml of toluene, was suspended 0.088 g (200 µmol) of a pyridinebisimine iron complex, [2,6-[(2,6-C₆H₃(i-C₃H₇)₂)N=C(Me)]₂C₅H₃N]FeCl₂, which was prepared according to the method described in J. Am. Chem. Soc., 1998, 4049 and Chem. Commun., 1998, 849, thereby preparing a complex slurry B (complex content: 10 µmol/ml). In a Schlenk tube, 5.0 ml of the co-catalyst solution F prepared in Example 8(1) and 0.2 ml of the complex slurry B were mixed and stirred for one hour at room temperature to prepare a catalyst solution H.
(2) Polymerization of ethylene
   Into a 1.6-liter autoclave of 50°C, 400 ml of dry cyclohexane, 0.5 ml of toluene solution of triisobutylaluminum (Al content: 1.0 mol/liter) and all of the catalyst solution H prepared above were successively added in nitrogen stream. Then, ethylene was pumped into the autoclave to keep the reaction pressure at 0.8 MPa·G. After 30 minutes of starting the introduction of ethylene, the supply of ethylene was stopped and the reaction liquid was rapidly cooled by cooling water. After cooling and pressure release, 22.8 g of polymer were obtained by filtration of the reaction mixture. However, no cyclohexane solubles and no oligomer were obtained. ¹³C-NMR measurement showed that the polymer obtained was a vinyl-terminated polymer. The polymerization activity for polyethylene was 410 kg/gFe/h.

### Example 10

(1) Preparation of co-catalyst solution I
   In the same manner as in Example 8(1) except for adding 25 ml of 0.5 mol/liter toluene solution of triisobutylaluminum (TIBA) at room temperature to 1.0 g of S-BEN (organic bentonite available from Hojun Yoko Co., Ltd.) placed in a 300-ml Schlenk tube, the co-catalyst solution I (content of clay-quaternary ammonium salt composite: 20 mg/ml) was prepared.
(2) Preparation of catalyst solution I
   In the same manner as in Example 8(2) except for using the co-catalyst solution I and the complex slurry A, the catalyst solution I was prepared.
(3) Polymerization of ethylene
   The polymerization of ethylene was conducted in the same manner as in Example 8(3) except for using the catalyst solution I instead of the catalyst solution F, thereby obtaining 132.8 g (total yield) of the reaction product containing 1.47 g of polymer and 131.33 g of cyclohexane solubles. The total polymerization activity and the polymerization activity for the cyclohexane solubles were 2380 kg/g Fe/h and 2350 kg/g Fe/h, respectively. ¹³C-NMR measurement showed that the polymer obtained was a vinyl-terminated polymer. Gas chromatographic analysis showed that the cyclohexane solubles contained 98 % by weight or more of a vinyl-terminated, linear α-olefin (oligomer having a carbon number of 6 to 18). In the gas chromatographic analysis, OV-1 column (60 m) was used for determining the amount of oligomer and Ultra-2 column (50 m) was used for determining the purity. Example 11

(1) Preparation of catalyst solution J
   A silylated compound was prepared by the reaction of trimethylchlorosilane and a reaction product of 2,6-dimethylaniline and 1,3-dibromopropane according to the method described in Macromolecules, 1996, 29, 5241. Then, the silylated compound was treated with titanium tetrachloride to synthesize a McConville-type titanium complex, [ArNCH₂CH₂CH₂NAr]TiCl₂, wherein Ar is 2,6-dimethylphenyl. The titanium complex thus obtained was suspended in heptane to prepare a complex slurry C (complex content: 10 µmol/ml). In a Schlenk tube, 5.0 ml of the co-catalyst solution F prepared in Example 8(1) and 1.0 ml of the complex slurry C were mixed and stirred for one hour at room temperature to prepare a catalyst solution J.
(2) Polymerization of ethylene
   Into a 1.6-liter autoclave of 80°C, 400 ml of dry cyclohexane, 0.5 ml of toluene solution of triisobutylaluminum (Al content: 1.0 mol/liter) and all of the catalyst solution J prepared above were successively added in nitrogen stream. Then, ethylene was pumped into the autoclave to keep the reaction pressure at 0.8 MPa·G. After one hour of starting the introduction of ethylene, the supply of ethylene was stopped and the reaction liquid was rapidly cooled by cooling water. After cooling and pressure release, the reaction liquid was filtered to obtain 5.3 g of polymer. However, no cyclohexane solubles and no oligomer were obtained. The polymerization activity for polyethylene was 11 kg/gTi/h.

### Comparative Example 3

(1) Polymerization of ethylene
The polymerization of ethylene was conducted in the same manner as in Example 11(2) except for using 0.5 ml of toluene solution of methylalumoxane (Al content: 1.98 mol/liter, available from Albemarle Co., Ltd.) and 1.0 ml of heptane solution of a McConville-type titanium complex, [ArNCH₂CH₂CH₂NAr]TiCl₂, wherein Ar is 2,6-dimethylphenyl, instead of 0.5 ml of toluene solution of triisobutylaluminum (Al content: 1.0 mol/liter) and the catalyst solution J. After one hour of the ethylene polymerization, 0.7 g of polymer was obtained. However, no cyclohexane solubles and no oligomer were obtained. The polymerization activity for polyethylene was 1.5 kg/gTi/h.

### Example 12

(1) Preparation of amine/silane/layered compound co-catalyst component K for polymerizing vinyl compounds and co-catalyst solution K
   Into a 2-liter flack containing one liter of distilled water, 2.5 g of Na-montmorillonite (BEN-GEL, available from Hojun Yoko, Co., Ltd.) were slowly added under stirring with a stirrer. After the addition was completed, stirring was continued for two hours at room temperature to prepare a colloidal solution. After heating the colloidal solution to 70°C, 1.0 g of diphenylsilyl dichloride (R²⁶ is phenyl, X is chlorine and r is 2 in the formula (7): R²⁶ᵣSiX₄₋ᵣ) dissolved in 10 ml of isopropanol was added over 10 minutes and stirring was further continued for 2.5 hours at the same temperature. The resultant slurry of the silane-treated layered compound was further added with an aqueous solution of 0.792 g (2 mmol) of benzylcetyldimethylammonium chloride (quaternary ammonium salt) dissolved in 100 ml of water. After the addition, the mixture was stirred for 30 minutes at the same temperature. The slurry thus obtained was filtered under heating through a pressure filter. The separated solid product was vacuum-dried at room temperature to obtain 3.5 g of the amine/silane/layered compound co-catalyst component K for polymerizing vinyl compounds.
   Into a 300-ml Schlenk tube, 1.0 g of the amine/silane/layered compound co-catalyst K for polymerizing vinyl compounds and 20 ml of toluene were placed, to which 25 ml of 0.5 mol/liter toluene solution of triisobutylaluminum (TIBA) were added at room temperature. The mixture was heated to 100°C and stirred at the same temperature for one hour. After cooling, the TIBA-treated slurry thus obtained was added with 250 ml of dry toluene and allowed to stand. The supernatant was discarded by a cannula and the slurry was washed with toluene repeatedly. Finally, the volume of the slurry was adjusted to 50 ml with toluene to obtain the co-catalyst solution K (content of co-catalyst component K for polymerizing vinyl compounds: 20 mg/ml).
(2) Preparation of catalyst solution K
   Into 20 ml of toluene, was suspended 0.088 g (200 µmol) of a pyridinebisimine iron complex, [2,6-[(2,4-Me₂C₆H₃)N=C(Me)]₂C₅H₃N]₂FeCl₂, which was prepared according to the method described in J. Am. Chem. Soc., 1998, 4049 and Chem. Commun., 1998, 849, thereby preparing a complex slurry A (complex content: 10 µmol/ml). In a Schlenk tube, 5.0 ml of the co-catalyst solution K and 0.2 ml of the complex slurry A were mixed and stirred for two hours at room temperature to prepare the catalyst solution K.
(3) Polymerization of ethylene
   Into a 1.6-liter autoclave of 50°C, 400 ml of dry cyclohexane, 0.5 ml of toluene solution of triisobutylaluminum (Al content: 1.0 mol/liter) and all of the catalyst solution K prepared above were successively added in nitrogen stream. Then, ethylene was pumped into the autoclave to keep the reaction pressure at 0.8 MPa·G. After 30 minutes of starting the introduction of ethylene, the supply of ethylene was stopped and the reaction liquid was rapidly cooled by cooling water. After cooling and pressure release, 163.3 g of cyclohexane solubles were obtained. Gas chromatographic analysis showed that the cyclohexane solubles contained 99 % by weight of a vinyl-terminated, linear α-olefin (oligomer having a carbon number of 6 to 18). In the gas chromatographic analysis, OV-1 column (60 m) was used for determining the amount of oligomer and Ultra-2 column (50 m) was used for determining the purity. The obtained amount of solid cyclohexane insolubles, i.e., polymer, was 0.96 g. ¹³C-NMR measurement of the polymer showed the presence of a terminal vinyl group. The total polymerization activity, the oligomerization activity and the by-production rate of polymer which was defined as a ratio of the polymer amount to the total amount of the product were 2920 kg/gFe/h, 2900 kg/gFe/h and 0.6 % by weight, respectively.

### Example 13

(1) Preparation of amine/silane/layered compound co-catalyst component L for polymerizing vinyl compounds and co-catalyst solution L
   The amine/silane/layered compound co-catalyst component L for polymerizing vinyl compounds was prepared in the same manner as in Example 12(1) except for using a solution of 0.574 g (2 mmol) of tribenzylamine (tertiary alkylamine) dissolved in 20 ml of ethanol and 0.2 ml of conc. hydrochloric acid (36% by weight concentration) instead of an aqueous solution containing 0.792 g (2 mmol) of benzylcetyldimethylammonium chloride (quaternary ammonium salt). Then, also in the same manner as in Example 12(1), the co-catalyst solution L (content of co-catalyst component L for polymerizing vinyl compounds: 20 mg/ml) was prepared from the co-catalyst component L for polymerizing vinyl compounds.
(2) Preparation of catalyst solution L
   Into 20 ml of toluene, was suspended 0.113 g (200 µmol) of a pyridinebisimine iron complex, [2,6-[(2-MeC₆H₄)N=C(Ph)]₂C₅H₃N]₂FeCl₂, which was prepared according to the method described in the following "Synthesis of pyridinebisimine iron complex", thereby preparing a complex slurry D (complex content: 10 µmol/ml). In a Schlenk tube, 5.0 ml of the co-catalyst solution L and 0.1 ml of the complex slurry D were mixed and stirred for two hours at room temperature to prepare a catalyst solution L. "Synthesis of pyridinebisimine iron complex"
   (i) Synthesis of 2,6-dibenzoylpyridine ligand precursor
      Into a 200-ml, two-necked round flask equipped with a reflux condenser, 12.2 g (91.5 ml) of AlCl₃ were introduced, and the atmosphere of the flask was replaced with nitrogen. To the flask, a benzene solution of 6.12 g (30.0 mmol) of 2,6-pyridinedicarbonyl dichloride dissolved in 50 ml of benzene was added. The mixture was heated and refluxed under stirring for 5 hours. After allowing the mixture to stand for cooling, AlCl₃ was deactivated by little-by-little addition of an aqueous solution of NaHCO₃. The resultant solution was extracted with toluene. The organic layer was dried over anhydrous MgSO₄ and then the solvent was distilled off. The product thus obtained was purified by a silica gel column chromatography (hexane/ethyl acetate = 10/1 by volume) to obtain 6.87 g of 2,6-dibenzoylpyridine ligand precursor in 80% yield.
   (ii) Synthesis of 2,6-dibenzoylpyridine-di(2-methylphenyl)imine ligand
      To 0.50 g (4.7 mmol) of o-toluidine dissolved in 10 ml of tetrahydrofuran (THF), 1.5 ml of 1.6 ml/liter hexane solution of n-BuLi (2.4 mmol) were added at -78°C, thereby forming Li salt of o-toluidine. After heated to room temperature, the mixture was slowly added dropwise to a solution containing 0.20 g (0.67 mmol) of 2,6-dibenzoylpyridine prepared in (i) and 10 ml of THF. After stirring for 30 minutes, the excess of Li salt was deactivated by methanol and the solvent was distilled away under reduced pressure. Distilled water and toluene were added to the residue, and the solvent was distilled away from the organic layer. Further, the excess of toluidine was distilled away by heating to 100°C under a reduced pressure of 1 mmHg, thereby obtaining 0.32 g of 2,6-dibenzoylpyridine-di(2-methylphenyl)imine ligand in 50% yield.
   (iii) Synthesis of complex
      To a dichloromethane solution of the ligand, an excessive amount of methanol solution of FeCl₂(H₂O)₄ was added. The complex was formed immediately to change the color of the solution into deep blue. By distilling away the solvent and extracting with dichloromethane, the pyridinebisimine iron complex, [2,6-[(2-MeC₆H₄)N=C(Ph)]₂C₅H₃N]FeCl₂, represented by the following formula was obtained. 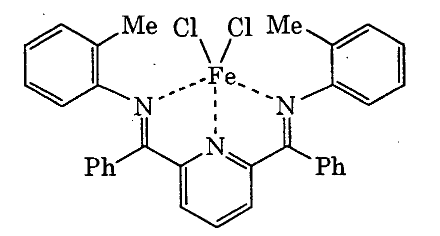
(3) Polymerization of ethylene
   The polymerization of ethylene was conducted in the same manner as in Example 12(3) except for using the catalyst solution L instead of the catalyst solution K. The amount of the total reaction product was 47.9 g and the amount of polymer was 1.55 g. The total polymerization activity, the oligomerization activity and the by-production rate of polymer were 1720 kg/gFe/h, 1660 kg/gFe/h and 3.2% by weight, respectively.
   Gas chromatographic analysis showed that the cyclohexane solubles contained 99% by weight of a vinyl-terminated, linear α-olefin (oligomer having a carbon number of 6 to 18). In the gas chromatographic analysis, OV-1 column (60 m) was used for determining the amount of oligomer and Ultra-2 column (50 m) was used for determining the purity. ¹³C-NMR measurement of the polymer showed the presence of a terminal vinyl group. Example 14

(1) Preparation of catalyst solution M
   Into 20 ml of toluene, was suspended 0.088 g (200 µmol) of a pyridinebisimine iron complex, [2,6-[(2,6-(i-C₃H₇)₂C₆H₃)N=C(Me)]₂C₅H₃N]FeCl₂, which was prepared according to the method described in J. Am. Chem. Soc., 1998, 4049 and Chem. Commun., 1998, 849, thereby preparing a complex slurry B (complex content: 10 µmol/ml). In a Schlenk tube, 5.0 ml of the co-catalyst solution K prepared in Example 12(1) and 0.2 ml of the complex slurry B were mixed and stirred for one hour at room temperature to prepare a catalyst solution M.
(2) Polymerization of ethylene
   The polymerization of ethylene was conducted for 30 minutes in the same manner as in Example 13(3) except for using the catalyst solution M instead of the catalyst solution L. Only polymer was obtained. By drying at 90°C for 8 hours under reduced pressure, 47.9 g of the product were obtained. The polymerization activity was 860 kg/gFe/h.

### Comparative Example 4

(1) Preparation of silane/layered compound co-catalyst component N for polymerizing vinyl compounds and co-catalyst solutionN
   Into a 2-liter flack containing one liter of distilled water, 2.5 g of Na-montmorillonite (BEN-GEL, available from Hojun Yoko, Co., Ltd.) were slowly added under stirring with a stirrer. After the addition was completed, stirring was continued for two hours at room temperature to prepare a colloidal solution. After heating the colloidal solution to 70°C, a solution of 1.0 g of diphenyldichlorosilane dissolved in 10 ml of isopropanol was added over 10 minutes. After the addition, the mixture was stirred for 3 hours at the same temperature. The slurry thus obtained was filtered under heating through a pressure filter. The separated solid product was vacuum-dried at room temperature to obtain 3.1 g of the silane/layered compound co-catalyst component N for polymerizing vinyl compounds.
   Then, into a 300-ml Schlenk tube, 1.0 g of the silane/layered compound co-catalyst component N for polymerizing vinyl compounds and 20 ml of toluene were placed, and the TIBA treatment was conducted in the same manner as in Example 12(1) to prepare the co-catalyst solution N (content of co-catalyst component N for polymerizing vinyl compounds: 20 mg/ml).
(2) Preparation of catalyst solution N
   The catalyst solution N was prepared by mixing 5.0 ml of the co-catalyst solution N prepared above and 0.2 mol of the complex slurry B prepared in Example 14(1) and stirring the resultant mixture for one hour at room temperature.
(3) Polymerization of ethylene
   The polymerization of ethylene was conducted in the same manner as in Example 14(2) except for using the catalyst solution N instead of the catalyst solution M. Only 18.4 g of polymer was obtained. The polymerization activity was 330 kg/gFe/h, which was about a half of Example 14.

### Example 15

(1) Preparation of amine/silane/layered compound co-catalyst component O for polymerizing vinyl compounds and co-catalyst solution O
   The amine/silane/layered compound co-catalyst component O for polymerizing vinyl compounds was prepared in the same manner as in Example 12(1) except for using 2.5 g of Na-montmorillonite (KUNIPIAF, available from Kunimine Kogyo Co., Ltd.) and 0.242 g (2 mmol) of dimethylaniline (tertiary alkylamine) instead of 2.5 g of Na-montmorillonite (BEN-GEL, available from Hojun Yoko, Co., Ltd.) and 0.792 g (2 mmol) of benzylcetyldimethylammonium chloride (quaternary ammonium salt). Then, also in the same manner as in Example 12(1), the co-catalyst solution O (content of co-catalyst component O for polymerizing vinyl compounds: 20 mg/ml) was prepared from the co-catalyst component O for polymerizing vinyl compounds.
(2) Preparation of catalyst solution O
   The catalyst solution O was prepared by mixing 0.1 ml of 10 µmol/ml n-heptane solution of dimethylsilylenebis(2-methyl-4,5-benzoindenyl) zirconium dichloride and 5.0 ml of the co-catalyst solution O, and stirring the resultant mixture for one hour at room temperature.
(3) Polymerization of propylene
   Into a 1.6-liter autoclave of 70°C, 400 ml of dry toluene, 0.5 ml of toluene solution of triisobutylaluminum (Al content: 1.0 mol/liter) and all of the catalyst solution O prepared above were successively added in nitrogen stream. Then, propylene was pumped into the autoclave to keep the reaction pressure at 0.5 MPa·G. After 30 minutes of starting the introduction of propylene, the supply of propylene was stopped and the reaction liquid was rapidly cooled by cooling water. After cooling and pressure release, the reaction product was filtered and dried at 90°C for 8 hours to obtain 39.4 g of polymer. The polymerization activity was 870 kg/gZr/h.

### Industrial Applicability

Since the catalyst for producing α-olefins of the present invention has a high oligomerization activity to ethylene, α-olefins can be efficiently produced with low costs. Also, vinyl-terminated, linear α-olefins (oligomers) having a molecular weight of 10000 or less or polyolefins having a molecular weight of larger than 10000 can be efficiently produced with low costs by using the catalyst of the present invention. The oligomers can be used as comonomers for olefin polymerization for producing LLDPE, etc. or materials for synthetic lubricant oils and cleaning agents.

## Claims

1. A catalyst for producing α-olefins, comprising (a) a complex of Group 8 to 10 transition metal of the Periodic Table, (b) clay, clay mineral or a ion-exchangeable layered compound, and (c) an amine compound or its adduct with Brφnsted acid.

2. A catalyst for producing α-olefins, comprising (a) a complex of Group 8 to 10 transition metal of the Periodic Table, (b) clay, clay mineral or a ion-exchangeable layered compound, (c) an amine compound or its adduct with Brφnsted acid, and (d) at least one organometallic compound selected from the group consisting of organoaluminum compounds, organomagnesium compounds, organolithium compounds and organozinc compounds.

3. The catalyst for producing α-olefins according to claim 1 or 2, wherein a ratio of the number of carbon atoms to the number of nitrogen atoms of the amine compound as the component (c) is 10 or more.

4. The catalyst for producing α-olefins according to any one of claims 1 to 3, wherein the component (c) is an amine compound having an aromatic ring or a hetero ring.

5. The catalyst for producing α-olefins according to any one of claims 1 to 4, wherein the component (a) is a transition metal complex represented by the formula (1):
L¹L²MX¹ ₘY¹ ₙ (1)
or the formula (2):
L¹L²L³MX¹ ₘY¹ ₙ (2)
wherein M is a Group 8 to 10 transition metal of the Periodic Table, L¹ to L³ may be bonded to each other to form a ring and are each independently a ligand capable of bonding to the transitional metal via a coordinating heteroatom, X¹ and Y¹ may be the same or different and are each independently a covalent- or ion-bonding group, m and n are each independently 0 or a positive integer and the sum of m and n is 0, 1, 2 or 3 depending on the valence of M.

6. The catalyst for producing α-olefins according to any one of claims 1 to 5, wherein the component (a) is a nitrogen-containing iron, cobalt or nickel chelate complex.

7. A process for producing α-olefins, comprising a step of oligomerizing ethylene in the presence of the catalyst for producing α-olefins as defined in any one of claims 1 to 6.

8. The process for producing α-olefins according to claim 7, wherein the oligomerization is carried out in an aliphatic hydrocarbon solvent.

9. A catalyst for polymerizing olefins, comprising (a) a chelate complex of Group 4 to 6 transition metal of the Periodic Table or a complex of Group 8 to 10 transition metal of the Periodic Table, (b) clay, clay mineral or a ion-exchangeable layered compound, and (c) a quaternary ammonium salt.

10. A catalyst for polymerizing olefins, comprising (a) a chelate complex of Group 4 to 6 transition metal of the Periodic Table or a complex of Group 8 to 10 transition metal of the Periodic Table, (b) clay, clay mineral or a ion-exchangeable layered compound, (c) a quaternary ammonium salt, and (d) at least one organometallic compound selected from the group consisting of organoaluminum compounds, organomagnesium compounds, organolithium compounds and organozinc compounds.

11. The catalyst for polymerizing olefins according to claim 9 or 10, wherein the component (a) is a transition metal complex represented by the formula (1):
L¹L²MX¹ ₘY¹ ₙ (1)
or the formula (2):
L¹L²L³MX¹ ₘY¹ ₙ (2)
wherein M is a Group 8 to 10 transition metal of the Periodic Table, L¹ to L³ may be bonded to each other to form a ring and are each independently a ligand capable of bonding to the transitional metal via a coordinating heteroatom, X¹ and Y¹ may be the same or different and are each independently a covalent- or ion-bonding group, m and n are each independently 0 or a positive integer and the sum of m and n is 0, 1, 2 or 3 depending on the valence of M.

12. The catalyst for polymerizing olefins according to any one of claims 9 to 11, wherein the component (a) is a chelate complex having a ligand coordinating to the transition metal via two or more nitrogen atoms.

13. The catalyst for polymerizing olefins according to any one of claims 9 to 12, wherein the component (a) is a nitrogen-containing iron, cobalt or nickel chelate complex.

14. The catalyst for polymerizing olefins according to any one of claims 9 to 13, wherein the component (c) is a quaternary ammonium salt in which a ratio of the number of carbon atoms to the number of nitrogen atoms is 8 or more.

15. The catalyst for polymerizing olefins according to any one of claims 9 to 14, wherein the component (c) has at least one aromatic ring-containing group, or two or more alkyl groups having 6 or more carbon atoms.

16. A process for polymerizing olefins, comprising a step of oligomerizing ethylene in the presence of the catalyst for polymerizing olefins as defined in any one of claims 9 to 15.

17. The process for polymerizing olefins according to claim 16, wherein the polymerization is carried out in an aliphatic hydrocarbon solvent.

18. A co-catalyst component for polymerizing vinyl compounds, produced by contacting (a) clay, clay mineral or a ion-exchangeable layered compound, (b) an amine compound, its adduct with Brφnsted acid or a quaternary ammonium salt, and (c) an organosilane compound.

19. The co-catalyst component for polymerizing vinyl compounds according to claim 18, wherein the component (a) is an ion-exchangeable, silicon-containing layered compound.

20. The co-catalyst component for polymerizing vinyl compounds according to claim 18 or 19 , wherein the component (a) is a clay mineral selected from the group consisting of smectite group minerals and mica group minerals.

21. The co-catalyst component for polymerizing vinyl compounds according to any one of claims 18 to 20, wherein the component (b) is a tertiary alkylamine, an adduct of the tertiary alkylamine and Brφnsted acid or a quaternary alkylammonium salt.

22. The co-catalyst component for polymerizing vinyl compounds according to any one of claims 18 to 21, wherein the component (b) is an amine compound in which a ratio of the number of carbon atoms to the number of nitrogen atoms is 8 or more.

23. The co-catalyst component for polymerizing vinyl compounds according to any one of claims 18 to 22, wherein the component (c) is an organosilane compound represented by the following formula:
R²⁶ ᵣSiX₄₋ᵣ
wherein R²⁶ is a group having a carbon or silicon atom which is directly bonded to Si or hydrogen, X is halogen or a group having an oxygen or nitrogen atom which is directly bonded to Si, r is an integer from 1 to 3, and a plurality of R²⁶ groups or X groups, if present, may be the same or different from each other.

24. The co-catalyst component for polymerizing vinyl compounds according to any one of claims 18 to 23, wherein R²⁶ is alkyl, benzyl or aromatic group and X is halogen or oxygen-containing group.

25. A catalyst for polymerizing vinyl compounds, comprising (d) the co-catalyst component for polymerizing vinyl compounds as defined in any one of claims 18 to 24, and (e) a complex of Group 4 to 6 transition metal of the Periodic Table or a complex of Group 8 to 10 transition metal of the Periodic Table.

26. A catalyst for polymerizing vinyl compounds, comprising (d) the co-catalyst component for polymerizing vinyl compounds as defined in any one of claims 18 to 24, (e) a complex of Group 4 to 6 transition metal of the Periodic Table or a complex of Group 8 to 10 transition metal of the Periodic Table, and (f) an organoaluminum compound.

27. The catalyst for polymerizing vinyl compounds according to claim 25 or 26, wherein the component (e) is a complex of Group 4 to 6 transition metal of the Periodic Table having a five-membered carbon ring or a complex of Group 4 to 6 or Group 8 to 10 transition metal of the Periodic Table having a ligand which coordinates the transition metal via a hetero atom.

28. The catalyst for polymerizing vinyl compounds according to any one of claims 25 to 27, wherein the transition metal of the component (e) is selected from the group consisting of titanium, zirconium, hafnium, vanadium, chromium, nickel, cobalt and iron.

29. A process for producing polymers of vinyl compounds, comprising a step of polymerizing at least one vinyl compound selected from the group consisting of olefins, styrene, styrene derivatives, acrylic derivatives and vinyl esters of fatty acids in the presence of the catalyst for polymerizing vinyl compounds as defined in any one of claims 25 to 28.

30. The process for producing polymers of vinyl compounds according to claim 29, wherein the vinyl compound is ethylene, propylene or styrene.

31. The process for producing polymers of vinyl compounds according to claim 29 or 30, wherein the polymerization of the vinyl compounds is carried out in a saturated hydrocarbon.

32. The process for producing polymers of vinyl compounds according to any one of claims 29 to 31, wherein the polymers of vinyl compounds are vinyl-terminated oligomers having a number average molecular weight of 10000 or less.
